(19) Europäisches Patentamt / European Patent Office / Office européen des brevets

(11) **EP 1 715 037 B1**

(12) **EUROPEAN PATENT SPECIFICATION**

(45) Date of publication and mention
of the grant of the patent:
**17.02.2010 Bulletin 2010/07**

(21) Application number: **04820173.5**

(22) Date of filing: **06.12.2004**

(51) Int Cl.:
*C12N 15/00* (2006.01)    *C12Q 1/68* (2006.01)

(86) International application number:
**PCT/JP2004/018137**

(87) International publication number:
**WO 2005/056790 (23.06.2005 Gazette 2005/25)**

(54) **METHOD OF AMPLIFYING NUCLEIC ACID**

VERFAHREN ZUR NUKLEINSÄUREAMPLIFIKATION

METHODE D'AMPLIFICATION D'ACIDE NUCLEIQUE

(84) Designated Contracting States:
**AT BE BG CH CY CZ DE DK EE ES FI FR GB GR HU IE IS IT LI LT LU MC NL PL PT RO SE SI SK TR**

(30) Priority: **10.12.2003 JP 2003412326**

(43) Date of publication of application:
**25.10.2006 Bulletin 2006/43**

(73) Proprietor: **TAKARA BIO INC.**
**Otsu-shi,**
**Shiga 520-2193 (JP)**

(72) Inventors:
- **UEMORI, Takashi**
  **Otsu-shi,**
  **Shiga 5202193 (JP)**
- **TAKEDA, Osamu**
  **Otsu-shi,**
  **Shiga 5202193 (JP)**
- **YAMAMOTO, Junko**
  **Otsu-shi,**
  **Shiga 5202193 (JP)**
- **MUKAI, Hiroyuki**
  **Otsu-shi,**
  **Shiga 5202193 (JP)**
- **ASADA, Kiyozo**
  **Otsu-shi,**
  **Shiga 5202193 (JP)**

- **KATO, Ikunoshin**
  **Otsu-shi,**
  **Shiga 5202193 (JP)**

(74) Representative: **Schnappauf, Georg et al**
**Dr. Volker Vossius**
**Patent- und Rechtsanwaltskanzlei**
**Geibelstrasse 6**
**81679 München (DE)**

(56) References cited:
**EP-A1- 1 312 682**    **WO-A1-00/28082**
**WO-A1-02/16639**    **JP-A- 2003 052 380**
**JP-A- 2005 052 002**

- **HAFNER G J ET AL: "ISOTHERMAL AMPLIFICATION AND MULTIMERIZATION OF DNA BY BST DNA POLYMERASE" BIOTECHNIQUES, INFORMA LIFE SCIENCES PUBLISHING, WESTBOROUGH, MA, US, vol. 30, no. 4, April 2001 (2001-04), pages 852-853,855, XP008011191 ISSN: 0736-6205**

Remarks:
The file contains technical information submitted after the application was filed and not included in this specification

SEQUENCE LISTING

<110> TAKARA BIO INC.

<120> A method for nucleic acid amplification

<130> 664878

<150> JP 2003-412326
<151> 2003-12-10

<160> 49

<170> PatentIn version 3.1

<210> 1
<211> 242
<212> DNA
<213> Artificial Sequence

<220>
<223> A portion of SARS coronavirus genomic RNA reverse transcribed to DNA. "nucleotide 1 to 5 is HindIII restriction site- nucleotide 238 to 242 is BamHI restriction site."

<400> 1

aagctttctc tatgatgggt ttcaaaatga attaccaagt caatggttac cctaatatgt    60

ttatcacccg cgaagaagct attcgtcacg ttcgtgcgtg gattggcttt gatgtagagg    120

gctgtcatgc aactagagat gctgtgggta ctaacctacc tctccagcta ggattttcta    180

caggtgttaa cttagtagct gtaccgactg gttatgttga cactgaaaat aacacaggat    240

cc                                      242

<210> 2
<211> 18
<212> DNA
<213> Artificial Sequence

<220>
<223> Designed chimeric oligonucleotide primer designated as 205RN3(1 8) for synthesizing cDNA from mRNA, and to amplify a portion of SARS coronavirus genome. "nucleotides 16 to 18 are ribonucleotide s- other nucleotides are deoxyribonucleotides."

<400> 2
agttgcatga cagcccuc          18

<210> 3
<211> 30
<212> DNA
<213> Artificial Sequence

<220>
<223> Designed oligonucleotide primer designated as A12-205R for synthesizing cDNA from mRNA.

<400> 3
aaacatatta ggagttgcat gacagccctc          30

<210> 4
<211> 18
<212> DNA
<213> Artificial Sequence

<220>

<223> Designed oligonucleotide primer designated as 215R for synthesizing cDNA from mRNA.

<400> 4
cagcatctct agttgcat          18

<210> 5
<211> 30
<212> DNA
<213> Artificial Sequence

<220>
<223> Designed oligonucleotide primer designated as A12-215R for synthesizing cDNA from mRNA, and to amplify a portion of SARS coronavirus genome.

<400> 5
aaacatatta ggcagcatct ctagttgcat          30

<210> 6
<211> 30
<212> DNA
<213> Artificial Sequence

<220>
<223> Designed oligonucleotide primer designated as A12-223R for synthesizing cDNA from mRNA, and to amplify a portion of SARS coronavirus genome.

<400> 6
aaacatatta ggagtaccca cagcatctct          30

<210> 7
<211> 18
<212> DNA
<213> Artificial Sequence

<220>
<223> Designed chimeric oligonucleotide primer designated as 134FN3(18) to amplify a portion of SARS coronavirus genome. "nucleotides 16 to 18 are ribonucleotides- other nucleotides are deoxyribonucleotides."

<400> 7
atcacccgcg aagaagcu          18

<210> 8
<211> 30
<212> DNA
<213> Artificial Sequence

<220>
<223> Designed oligonucleotide primer designated as A12(-10)-215R for synthesizing cDNA from mRNA, and to amplify a portion of SARS coronavirus genome.

<400> 8
gggtaaccat tgcagcatct ctagttgcat          30

<210> 9
<211> 30
<212> DNA
<213> Artificial Sequence

<220>
<223> Designed oligonucleotide primer designated as A12(-20)-215R for synthesizing cDNA from mRNA, and to amplify a portion of SARS coronavirus genome.

<400> 9
tgacttggta atcagcatct ctagttgcat          30

<210> 10
<211> 30
<212> DNA
<213> Artificial Sequence

<220>
<223> Designed oligonucleotide primer designated as A12(6)-215R for synthesizing cDNA from mRNA, and to amplify a portion of SARS coronavirus genome.

<400> 10
ggtgataaac atcagcatct ctagttgcat          30

<210> 11
<211> 30
<212> DNA
<213> Artificial Sequence

<220>
<223> Designed oligonucleotide primer designated as A12(12)-215R for synthesizing cDNA from mRNA, and to amplify a portion of SARS coronavirus genome.

<400> 11
ttcgcgggtg atcagcatct ctagttgcat          30

<210> 12
<211> 16
<212> DNA
<213> Artificial Sequence

<220>
<223> Designed chimeric oligonucleotide primer designated as B134FN3( 16) to amplify a portion of SARS coronavirus genome. "nucleotides 14 to 16 are ribonucleotides- other nucleotides are deoxyribonucleotides." "5'-end is labeled with biotin."

<400> 12
atcacccgcg aagaag          16

<210> 13
<211> 16
<212> DNA
<213> Artificial Sequence

<220> <223> Designed chimeric oligonucleotide primer designated as 205RN3(16) to amplify a portion of SARS coronavirus genome. "nucleotides 14 to 16 are ribonucleotides- other nucleotides are deoxyribonucleotides."

<400> 13
agttgcatga cagccc          16
<210> 14
<211> 24
<212> DNA
<213> Artificial Sequence

<220>
<223> Designed oligonucleotide primer designated as A6(-10)-215R for synthesizing cDNA from mRNA, and to amplify a portion of SARS coronavirus genome.

<400> 14
gggtaacagc atctctagtt gcat          24

<210> 15
<211> 27
<212> DNA
<213> Artificial Sequence

<220>
<223> Designed oligonucleotide primer designated as A9(-10)-215R for synthesizing cDNA from mRNA, and to amplify a portion of SARS coronavirus genome.

<400> 15
gggtaaccac agcatctcta gttgcat          27

<210> 16
<211> 20
<212> DNA
<213> Artificial Sequence

<220>
<223> Designed oligonucleotide probe designated as SARS-BNI-B for detecting an amplified a portion of SARS coronavirus genome. "5'-end is labeled with FITC."

<400> 16
aagccaatcc acgcacgaac          20

<210> 17
<211> 18
<212> DNA
<213> Artificial Sequence

<220>
<223> Designed chimeric oligonucleotide primer designated as 160FN3 to amplify a portion of SARS coronavirus genome. "nucleotides 16 to 18 are ribonucleotides- other nucleotides are deoxyribonucleot ides."

<400> 17
cgttcgtgcg tggatugg          18

<210> 18
<211> 14
<212> DNA
<213> Artificial Sequence

<220>
<223> Designed chimeric oligonucleotide primer designated as 241RN3 to amplify a portion of SARS coronavirus genome. "nucleotides 12 to 14 are ribonucleotides- other nucleotides are deoxyribonucleotides."

<400> 18
tagctggaga ggua          14

<210> 19
<211> 21
<212> DNA

<213> Artificial Sequence

<220>
<223> Designed chimeric oligonucleotide primer designated as (A12)241 RN3 to amplify a portion of SARS coronavirus genome. "nucleotides 18 to 21 are ribonucleotides- other nucleotides are deoxyribonucleotides."

<400> 19
tgacgaatag ctggagaggu a        21

<210> 20
<211> 16
<212> DNA
<213> Artificial Sequence

<220>
<223> Designed chimeric oligonucleotide primer designated as 134FN3(16) to amplify a portion of SARS coronavirus genome. "nucleotides 14 to 16 are ribonucleotides- other nucleotides are deoxyribonucleotides."

<400> 20
atcacccgcg aagaag        16

<210> 21
<211> 20
<212> DNA
<213> Artificial Sequence

<220>
<223> Designed chimeric oligonucleotide primer designated as ICAN-ALD H2-F to amplify a portion of human aldehyde dehydrogenase 2 gene. "nucleotides 18 to 20 are ribonucleotides- other nucleotides are deoxyribonucleotides."

<400> 21
agttgggcga gtacgggcug        20

<210> 22
<211> 20
<212> DNA
<213> Artificial Sequence

<220>
<223> Designed chimeric oligonucleotide primer designated as ICAN-ALD H2-R to amplify a portion of human aldehyde dehydrogenase 2 gene. "nucleotides 18 to 20 are ribonucleotides- other nucleotides are deoxyribonucleotides."

<400> 22
cagaccctca agccccaaca        20

<210> 23
<211> 14
<212> DNA
<213> Artificial Sequence

<220>
<223> Designed chimeric oligonucleotide probe designated as ALDH2 wG probe for detecting an amplified a portion of native human aldehyde dehydrogenase 2 gene. "nucleotides 11 is ribonucleotide- other nucleotides are deoxyribonucleotides." "5'-end is labeled with ROX, and 3'-end is labeled with Eclipse."

<400> 23

ggcatacact gaag          14

<210> 24
<211> 14
<212> DNA
<213> Artificial Sequence

<220>
<223> Designed chimeric oligonucleotide probe designated as ALDH2 mA probe for detecting an amplified a portion of mutant human aldehyde dehydrogenase 2 gene. "nucleotides 11 is ribonucleotide- other nucleotides are deoxyribonucleotides." "5'-end is labeled with FAM, and 3'-end is labeled with Eclipse."

<400> 24
ggcatacact aaag          14

<210> 25
<211> 29
<212> DNA
<213> Artificial Sequence

<220>
<223> Designed oligonucleotide primer designated as ALDH2-TH1 to amplify a portion of human aldehyde dehydrogenase 2 gene.

<400> 25
cccggccact ccgcagaccc tcaagcccc          29

<210> 26
<211> 28
<212> DNA
<213> Artificial Sequence

<220>
<223> Designed oligonucleotide primer designated as ALDH2-TH2 to amplify a portion of human aldehyde dehydrogenase 2 gene.

<400> 26
cccggccact ccagccacca gcagaccc          28

<210> 27
<211> 28
<212> DNA
<213> Artificial Sequence

<220>
<223> Designed oligonucleotide primer designated as ALDH2-TH3 to amplify a portion of human aldehyde dehydrogenase 2 gene.

<400> 27
cccggccact ccaggctccg agccacca          28

<210> 28
<211> 21
<212> DNA
<213> Artificial Sequence

<220>
<223> Designed oligonucleotide PCR primer designated as ALDH2-F to amplify a portion of human aldehyde dehydrogenase 2 gene.

<400> 28
cagggtcaac tgctatgatg t          21

<210> 29
<211> 21
<212> DNA
<213> Artificial Sequence

<220>
<223> Designed oligonucleotide PCR primer designated as ALDH2-R to amplify a portion of human aldehyde dehydrogenase 2 gene.

<400> 29
agcccccaac agaccccaat c          21

<210> 30
<211> 16
<212> DNA
<213> Artificial Sequence

<220>
<223> Designed oligonucleotide primer designated as ALDH2-TH4 to amplify a portion of human aldehyde dehydrogenase 2 gene.

<400> 30
agccaccagc agaccc          16

<210> 31
<211> 17
<212> DNA
<213> Artificial Sequence

<220>
<223> Designed chimeric oligonucleotide primer designated as F2 to amplify a portion of Legionella pneumophila mip gene. "nucleotides 15 to 17 are ribonucleotides- other nucleotides are deoxyribonucleotides."

<400> 31
atggggcttg caatguc          17

<210> 32
<211> 17
<212> DNA
<213> Artificial Sequence

<220>
<223> Designed chimeric oligonucleotide primer designated as R2 to amplify a portion of Legionella pneumophila mip gene. "nucleotides 15 to 17 are ribonucleotides- other nucleotides are deoxyribonucleotides."

<400> 32
agtagctaat gatgugg          17

<210> 33
<211> 12
<212> DNA
<213> Artificial Sequence

<220>
<223> Designed chimeric oligonucleotide probe designated as Mip4g12 probefor detecting an amplified a portion of Legionella pneumophi a mip gene. "nucleotides 4 is ribonucleotide- other nucleotides are deoxyribonucleotides." "5'-end is labeled with FAM, and 3'-end is labeled with Eclipse."

<400> 33
aatggctgca ac        12


<210> 34
<211> 17
<212> DNA
<213> Artificial Sequence


<220>
<223> Designed oligonucleotide primer designated as R2(-13) to amplify a portion of Legionella pneumophila mip gene.

<400> 34
ccaatgctat aagacaa        17


<210> 35
<211> 29
<212> DNA
<213> Artificial Sequence


<220>
<223> Designed oligonucleotide primer designated as R2(-13)A12-1 to amplify a portion of Legionella pneumophila mip gene.

<400> 35
aacagctgca gtccaatgct ataagacaa        29


<210> 36
<211> 29
<212> DNA
<213> Artificial Sequence


<220>
<223> Designed oligonucleotide primer designated as R2(-13)A12-2 to amplify a portion of Legionella pneumophila mip gene.

<400> 36
caccaatttc atccaatgct ataagacaa        29

<210> 37
<211> 20
<212> DNA
<213> Artificial Sequence


<220>
<223> Designed oligonucleotide PCR primer designated as c-Ki-ras/12F to amplify a portion of human c-Ki-ras2 gene.

<400> 37
gactgaatat aaacttgtgg        20


<210> 38
<211> 23
<212> DNA

<213> Artificial Sequence

<220>
<223> Designed oligonucleotide PCR primer designated as rasT1R to amplify a portion of human c-Ki-ras2 gene.

<400> 38
aaactattgt tggatcatat tcg          23

<210> 39
<211> 25
<212> DNA
<213> Artificial Sequence

<220>
<223> Designed oligonucleotide PCR primer designated as rasT14F to amplify a portion of human c-Ki-ras2 gene.

<400> 39
gcgcggactg aatataaact tgtgg          25

<210> 40
<211> 29
<212> DNA
<213> Artificial Sequence

<220>
<223> Designed oligonucleotide PCR primer designated as rasT4R to amplify a portion of human c-Ki-ras2 gene.

<400> 40
aaacgcgcgc tattgttgga tcatattcg          29

<210> 41
<211> 20
<212> DNA
<213> Artificial Sequence

<220>
<223> Designed chimeric oligonucleotide primer designated as c-Ki-ras /12FN3 to amplify a portion of human c-Ki-ras2 gene. "nucleotide s 18 to 20 are ribonucleotide- other nucleotides are deoxyribonucleotides."

<400> 41
gactgaatat aaacttgugg          20

<210> 42
<211> 20
<212> DNA
<213> Artificial Sequence

<220>
<223> Designed chimeric oligonucleotide primer designated as c-Ki-ras /12RN3 to amplify a portion of human c-Ki-ras2 gene. "nucleotide s 18 to 20 are ribonucleotide- other nucleotides are deoxyribonucleotides."

<400> 42
ctattgttgg atcatatucg          20
<210> 43
<211> 20
<212> DNA
<213> Artificial Sequence

<220>
<223> Designed oligonucleotide primer designated as PJDBF to amplify a portion of Neisseria gonorrhoeae cppB gene.

<400> 43
ctttgcttca atgcctcgtt        20

<210> 44
<211> 17
<212> DNA
<213> Artificial Sequence

<220>
<223> Designed oligonucleotide primer designated as PJDBR to amplify a portion of Neisseria gonorrhoeae cppB gene.

<400> 44
catcacgcac cgaagcc        17

<210> 45
<211> 20
<212> DNA
<213> Artificial Sequence

<220>
<223> Designed chimeric oligonucleotide primer designated as PJDB0FN3 to amplify a portion of Neisseria gonorrhoeae cppB gene. "nucleotides 18 to 20 are ribonucleotide- other nucleotides are deoxyribonucleotides."

<400> 45
ctttgcttca atgcctcguu        20

<210> 46
<211> 17
<212> DNA
<213> Artificial Sequence

<220>
<223> Designed chimeric oligonucleotide primer designated as PJDB0RN3 to amplify a portion of Neisseria gonorrhoeae cppB gene. "nucleotides 15 to 17 are ribonucleotide- other nucleotides are deoxyribonucleotides."

<400> 46
catcacgcac cgaagcc        17

<210> 47
<211> 24
<212> DNA
<213> Artificial

<220>
<223> Designed oligonucleotide primer designated as A6-215R to amplify a portion of SARS coronavirus genome.

<400> 47
aaacatcagc atctctagtt gcat        24

<210> 48
<211> 27
<212> DNA
<213> Artificial

<220>
<223> Designed oligonucleotide primer designated as A9-215R to amplify a portion of SARS coronavirus genome.

<400> 48
aaacatattc agcatctcta gttgcat        27
<210> 49
<211> 36
<212> DNA
<213> Artificial

<220>
<223> Designed oligonucleotide primer designated as A18-205R to amplify a portion of SARS coronavirus genome.

<400> 49
aaacatatta gggtaaccag ttgcatgaca gccctc        36

**Description**

Technical Field

The present invention relates to a method for detecting a target nucleic acid which is useful in the field of clinical medicine, and a method for synthesizing a DNA which is useful in the field of genetic engineering. The present invention relates to a method for amplifying a nucleic acid as a template and a method for detecting a target nucleic acid amplified according to said method.

Background Art

DNA synthesis is used for various purposes in studies in the field of genetic engineering. Most of the DNA syntheses with the exception of those of short-chain DNAs (e.g., oligonucleotides) are carried out using an enzymatic method in which a DNA polymerase is utilized.
For example, according to the polymerase chain reaction (PCR) method, it is required to repeat reactions at a high temperature and a low temperature several times in order to regenerate single-stranded target molecules for the subsequent amplification cycles. Since the reactions are restricted by temperature as described above, the reaction system needs to be conducted using discontinuous phases or cycles.
Accordingly, this method requires the use of an expensive thermal cycler that can strictly adjust a wide range of temperatures over time. Furthermore, the reaction requires time for adjusting the temperature to the two or three predetermined ones. The loss of time increases in proportion to the cycle number.
Then, nucleic acid amplification methods that can be carried out under isothermal conditions have been developed in order to solve the above-mentioned problems.
Examples of the methods that can be carried out under isothermal conditions include the Strand Displacement Amplification (SDA) method (see, for example, Patent Document 1), the Rolling Circle Amplification (RCA) method (see, for example, Patent Document 2), the Loop-mediated isothermal AMPlification (LAMP) method (see, for example, Patent Document 3), the Isothermal and Chimeric primer-initiated Amplification of Nucleic acids (ICAN) method (see, for example, Patent Document 4) and the various modified SDA methods (see, for example, Patent Documents 5-8).
In the reaction of such an isothermal method of nucleic acid amplification or oligonucleotide synthesis, extension from a primer and annealing of a primer to a single-stranded extension product (or to an original target sequence) followed by extension from the primer take place in parallel in a reaction mixture incubated at a constant temperature.
The SDA method as described in Patent Document 1 is a method for amplifying a target nucleic acid sequence (and a complementary strand thereof) in a sample by displacement of double strands with the aid of a DNA polymerase and a restriction endonuclease. This method requires four primers used for amplification, and two of them need to be constructed to contain a recognition site for the restriction endonuclease. The method requires the use of a modified deoxyribonucleotide triphosphate as a substrate for DNA synthesis in large quantities. The modified deoxyribonucleotide triphosphate is exemplified by ($\alpha$-S) deoxyribonucleotide triphosphate in which an oxygen atom in the phosphate group at the $\alpha$-position is replaced by a sulfur atom (S).
The RCA method as described in Patent Document 2 is a nucleic acid amplification method in which a circular DNA is used as a template. The resulting amplification product has a sequence in which amplified regions are repeated several times, and serves as a new template. Consequently, the final amplification product has a branched structure called "branching". Regarding the RCA method, it has been reported that suppression of generation of a ladder-like amplification product in a side reaction is important for increasing the amplification efficiency (see, for example, Non-patent Document 1).
The LAMP method as described in Patent Document 3 requires four primers for amplification. The resulting amplification products are ladder-like DNAs with varying sizes in which target regions to be amplified are repeated. In the DNA, the target regions are connected.to each other in alternate directions.
The ICAN method as described in Patent Document 4 is a nucleic acid amplification method in which a chimeric oligonucleotide primer is used.
The modified SDA method as described in Patent Document 5 is a DNA amplification method in which a chimeric oligonucleotide primer is used. The chimeric oligonucleotide primer is composed of RNA and DNA and has, as an essential element, a structure in which DNA is positioned at least at the 3' terminus.
The modified SDA method as described in Patent Document 6 requires the use of a restriction enzyme that generates a 3'-protruding terminus.
The modified SDA method as described in Patent Document 7 requires the use of at least two pairs of primers.
The modified SDA method as described in Patent Document 8 requires the use of at least two pairs of primers and at least one modified deoxyribonucleotide triphosphate.
Isothermal nucleic acid amplification methods have been developed as described above, although these methods also have problems concerning the amplification efficiencies, the detection sensitivities and the like. Then, improved isothermal nucleic acid amplification methods have been developed in order to solve the problems (see, for example, Patent Documents 9-11).

The method as described in Patent Document 9 is a nucleic acid amplification method using the following: a primer that has a promoter for an RNA polymerase or a part thereof attached at the 5' terminus of a reverse transcription primer or a primer for nucleic acid amplification, and further has an arbitrary oligonucleotide attached at the 5' terminus which contains at least five nucleotides; and a third primer that anneals to an upstream region or a portion of the promoter for an RNA polymerase in the primer.

The method as described in Patent Document 10 is a nucleic acid amplification method in which at least two complementary primers or at least two primer pairs are used.
This method is a method in which the amount of generated amplification product is increased by the use of plural primers or plural primer pairs. It is necessary to use more primers or primer pairs for increasing the productivity.

The method as described in Patent Document 11 is a method according to the method as described in Patent Document 12 using the following in combination: a chimeric oligonucleotide primer; and an upstream oligonucleotide primer which hybridizes to a portion in a template nucleic acid 3' to the position at which the primer hybridizes.
According to this method, a ladder-forming oligonucleotide primer consists only of a sequence that hybridizes to a template strand.

The conventional isothermal nucleic acid amplification methods as described above still have various problems. Thus, an efficient and highly sensitive method has been desired.

Patent Document 1: JP-B 7-114718
Patent Document 2: WO 97/19193
Patent Document 3: WO 00/28082
Patent Document 4: WO 00/56877
Patent Document 5: US 5,824,517
Patent Document 6: WO 99/09211
Patent Document 7: WO 95/25180
Patent Document 8: WO 99/49081
Patent Document 9: WO 95/03426
Patent Document 10: WO 95/25180
Patent Document 11: JP-A 2003-52380
Non-patent Document 1: Hafner, G.J. et al., BioTechniques, vol.30 (2001) p.852-867

Disclosure of Invention

Problems to be Solved by the Invention

The main object of the present invention is to provide a method for amplifying a target nucleic acid by which a target nucleic acid in a sample is specifically amplified with high sensitivity.

Means to Solve the Problems

The present inventors have studied intensively in order to solve the above-mentioned problems. As a result, the present inventors have found that a ladder-like product is generated in an ICAN reaction if substantially identical sequences are present in a nucleic acid as a template, and a primer is designed for a sequence within a region between the sequences. In the ladder-like product, target regions are polymerized through the identical sequences in one direction. The present inventors have further found that the sensitivity, the amplification efficiency and the reaction velocity of an ICAN reaction can be increased by positively converting the amplification products into ladder-like ones. The conversion is accomplished by using a chimeric oligonucleotide primer and a ladder-forming oligonucleotide primer which contains a specific nucleotide sequence. Thus, the present invention has been completed.

The first aspect of the present invention relates to a method for amplifying a nucleic acid, the method comprising the steps of:

(A) preparing a reaction mixture selected from:

(a) a nucleic acid as a template, a deoxyribonucleotide triphosphate, a DNA polymerase having a strand displacement activity, at least two chimeric oligonucleotide primers, at least one ladder-forming oligonucleotide primer and an RNase H; or

(b) a nucleic acid as a template, a deoxyribonucleotide triphosphate, a DNA polymerase having a strand displacement activity, at least two chimeric oligonucleotide primers and an RNase H, wherein one of the chimeric oligonucleotide primers serves as a ladder-forming oligonucleotide primer,

wherein each chimeric oligonucleotide primer contains a ribonucleotide as well as at least one selected from the group consisting of a deoxyribonucleotide and a nucleotide analog, and the ribonucleotide is positioned at the 3' terminus or on the 3'-terminal side of the primer,

wherein the chimeric oligonucleotide primers comprise at least a first chimeric oligonucleotide primer which is complementary to a nucleotide sequence of the nucleic acid as a template and a second chimeric oligonucleotide primer which is homologous to a nucleotide sequence of the nucleic acid as a template, and

wherein the ladder-forming oligonucleotide primer has a sequence complementary to a region of the nucleic acid as a template that is complementary to the first chimeric oligonucleotide primer and/or a nucleotide sequence 3' to said region, and has, on its 5' side, a sequence complementary to: a nucleotide sequence on the 5' side of the second chimeric oligonucleotide primer which is homologous to the nucleic acid as a template; a nucleotide sequence of the nucleic acid as a template corresponding to a region 5' to the 5' terminus of the portion homologous to the second chimeric oligonucleotide primer; or both; and

(B) incubating the reaction mixture for a sufficient time to generate a ladder-like amplification product under constant-temperature conditions under which specific annealing of the primer to the nucleic acid as a template, a reaction of synthesizing an extended strand and a strand displacement reaction by the DNA polymerase, as well as a reaction of cleaving an extended strand by the RNase H take place.

According to the first aspect, the nucleic acid as a template may be an RNA, and the nucleic acid may be treated beforehand with a deoxyribonucleotide triphosphate, a DNA polymerase having a reverse transcription activity and at least one ladder-forming oligonucleotide primer to convert the nucleic acid into a reverse transcription product.

According to the first aspect, the reaction mixture in step (A) may further contain a DNA polymerase having a reverse transcription activity.

According to the first aspect, the nucleic acid as a template may be an mRNA.

According to the first aspect, a single DNA polymerase having a reverse transcription activity and a strand displacement activity may be used.

The second aspect of the present invention relates to a method for detecting a target nucleic acid, the method comprising the steps of:

(a) amplifying a target nucleic acid according to the method of the first aspect; and
(b) detecting the target nucleic acid amplified in the above step.

Effects of the Invention

The present invention provides an amplification method superior to conventional isothermal nucleic acid amplification methods.

Brief Description of Drawings

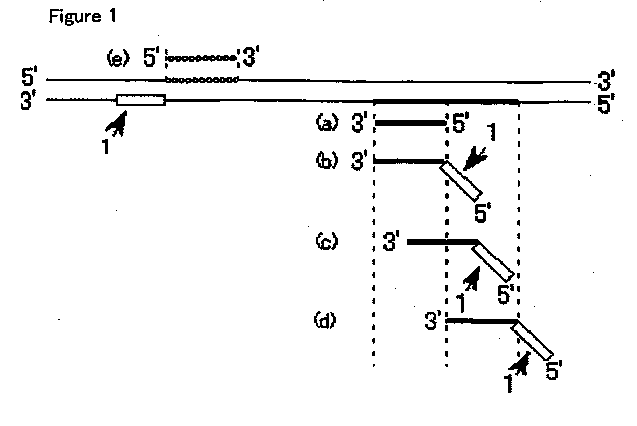

Figure 1 illustrates positional relationships among the first chimeric oligonucleotide primer, the second oligonucleotide primer and the ladder-forming oligonucleotide primers used in Example 2-(1)-(A). (a) represents the first chimeric oligonucleotide primer, (b) to (d) represent the ladder-forming oligonucleotide primers, and (e) represents the second chimeric oligonucleotide primer. Each of the parts 1 in (b) to (d) has a sequence homologous to the part 1 in the complementary strand of a portion 5' upstream of (e).

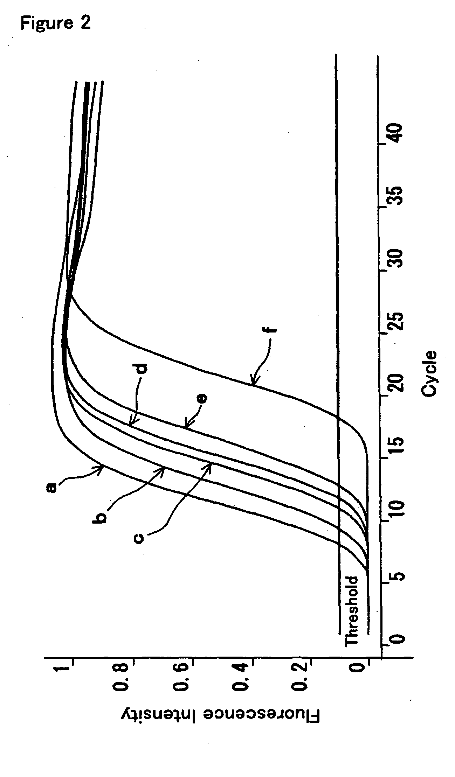

Figure 2 illustrates amplification curves for varying copy numbers. The horizontal axis in Figure 2 represents the reaction cycle number. In Figure 2, a, b, c, d, e and f represent amplification curves obtained using $10^5$ copies, $10^4$ copies, $10^3$ copies, $10^2$ copies, $10^1$ copies and $10^0$ copy of the template, respectively.

Best Mode for Carrying Out the Invention

As used herein, a deoxyribonucleotide (also referred to as a dN) refers to a nucleotide of which the sugar portion is composed of D-2-deoxyribose. Examples thereof include ones having adenine, cytosine, guanine or thymine as the base portion. Furthermore, the deoxyribonucleotides also include a deoxyribonucleotide having a modified base such as 7-deazaguanosine, a deoxyribonucleotide having a functional group that can be used for immobilization and a deoxyribonucleotide analog such as deoxyinosine nucleotide.

As used herein, a ribonucleotide (also referred to as an N) refers to a nucleotide of which the sugar portion is composed of D-ribose. The ribonucleotides include ones having adenine, cytosine, guanine or uracil as the base portion. The ribonucleotides also include modified ribonucleotides such as a modified ribonucleotide in which an oxygen atom in the phosphate group at the $\alpha$-position is replaced by a sulfur atom (S) (also referred to as an ($\alpha$-S) ribonucleotide or an ($\alpha$-S) N) or other derivatives.

As used herein, a chimeric oligonucleotide primer refers to an oligonucleotide primer that has a ribonucleotide positioned at the 3' terminus or on the 3'-terminal side of the primer, can be used to extend a nucleic acid strand according to the method of the present invention, can be cleaved with an RNase H, and can be used to effect a strand displacement reaction. The chimeric oligonucleotide primers used according to the present invention include any one having the above-mentioned constitution.

A chimeric oligonucleotide primer used according to the present invention contains a ribonucleotide as well as at least one selected from the group consisting of a deoxyribonucleotide and a nucleotide analog. The primers include oligoribonucleotide primers containing an unmodified ribonucleotide and/or a modified ribonucleotide.

A chimeric oligonucleotide primer used in the method of the present invention has a nucleotide sequence substantially complementary to a part of the nucleotide sequence of a nucleic acid as a template. As used herein, "a substantially complementary nucleotide sequence" means a nucleotide sequence that is capable of annealing to a DNA as a template under reaction conditions used.

As used herein, 3'-terminal side refers to a portion from the center to the 3' terminus of a nucleic acid (e.g., a primer). Likewise, 5'-terminal side refers to a portion from the center to the 5' terminus of a nucleic acid.

As used herein, an upstream region refers to a region of a template strand that is 5' to the 5' terminus of a nucleotide sequence of a chimeric oligonucleotide primer, wherein the template strand is homologous to the chimeric oligonucleotide primer to be used. Thus, it corresponds to a region of the strand complementary to the template strand that is 3' to the 3' terminus of the sequence complementary to the nucleotide sequence of the chimeric oligonucleotide primer.

As used herein, a nucleotide sequence for ladder formation refers to a nucleotide sequence complementary to: a nucleotide sequence on the 5' side of a chimeric oligonucleotide primer used in the method of the present invention; a nucleotide sequence of a nucleic acid as a template corresponding to a region upstream of (i.e., 5' to) the 5' terminus of a portion homologous to the chimeric oligonucleotide primer; or both.

As used herein, a ladder-forming oligonucleotide primer refers to one containing the above-mentioned nucleotide sequence for ladder formation on the 5' side of the primer.

Positions in a nucleotide sequence are indicated herein as follows: the 5' terminus of a chimeric oligonucleotide primer used in the method of the present invention or a position corresponding to the position in the template strand to which the chimeric oligonucleotide primer anneals is defined as "0". The positions towards the 3' terminus of the chimeric oligonucleotide primer are represented by integers "+1", "+2", ... The position 1 nucleotide upstream of the position "0" in the nucleic acid as a template is defined as "-1". The positions towards upstream are represented by integers "-2", "-3", ...

As used herein, an RNase H (ribonuclease H) may be any one that acts on a double-stranded DNA generated by DNA extension from a chimeric oligonucleotide primer that has been annealed to a nucleic acid as a template, and specifically cleaves it at a ribonucleotide portion in the primer.

As used herein, a DNA polymerase refers to an enzyme that synthesizes a new DNA strand using another DNA strand as a template (a DNA-dependent DNA polymerase) or an enzyme that synthesizes a DNA strand complementary to an RNA strand using the RNA strand as a template (an RNA-dependent DNA polymerase). The DNA polymerases include naturally occurring DNA polymerases and variant enzymes each having the above-mentioned activity. For example, such enzymes include a DNA polymerase having a strand displacement activity, a DNA polymerase lacking a 5'→3' exonuclease activity and a DNA polymerase additionally having a reverse transcriptase activity or an RNase H activity.

As used herein, "a strand displacement activity" refers to an activity that is capable of effecting a strand displacement, that is, that can proceed with DNA duplication on the basis of a sequence of a nucleic acid as a template while displacing a DNA strand to release the complementary strand that has been annealed to the template strand. In addition, the DNA strand released from the nucleic acid as a template as a result of strand displacement is referred to as "a displaced strand" herein.

As used herein, "a reverse transcription activity" refers to an activity that is capable of synthesizing a DNA strand complementary to an RNA strand using the RNA strand as a template.

(1) The method for amplifying a target nucleic acid of the present invention

It is possible to carry out the method of the present invention using at least one chimeric oligonucleotide primer and at least one ladder-forming oligonucleotide primer in combination with an RNase H and a DNA polymerase. Alternatively, it is possible to use a DNA polymerase having an RNase H activity under conditions under which it exhibits its RNase H activity.

A nucleic acid is successively amplified under isothermal conditions according to the method of the present invention. "Successively" means that a reaction proceeds without a change in the reaction temperature or the composition of the reaction mixture. As used herein, "isothermal" means conditions of a substantially constant temperature under which an enzyme and a nucleic acid strand function in each step as described below.

In one embodiment of the method for amplifying a target nucleic acid of the present invention, the method comprises the steps of:

(a) treating a nucleic acid as a template with a deoxyribonucleotide triphosphate, a DNA polymerase having a reverse transcription activity and at least one ladder-forming oligonucleotide primer to obtain a reverse transcription product;
(b) preparing a reaction mixture by mixing the reverse transcription product, a DNA polymerase having a strand displacement activity, at least two chimeric oligonucleotide primers and an RNase H; and
(c) incubating the reaction mixture for a sufficient time to generate a ladder-like amplification product under constant-temperature conditions under which a reaction of synthesizing an extended strand and a strand displacement reaction by the DNA polymerase, as well as a reaction of cleaving an extended strand by the RNase H take place.

Each chimeric oligonucleotide primer contains a ribonucleotide as well as at least one selected from the group consisting of a deoxyribonucleotide and a nucleotide analog, and the ribonucleotide is positioned at the 3' terminus or on the 3'-terminal side of the primer.

The chimeric oligonucleotide primers comprise at least a first chimeric oligonucleotide primer which is complementary to a nucleotide sequence of the nucleic acid as a template and a second chimeric oligonucleotide primer which is homologous to a nucleotide sequence of the nucleic acid as a template.

The ladder-forming oligonucleotide primer is complementary to a region of the nucleic acid as a template that is complementary to the first chimeric oligonucleotide primer and a nucleotide sequence 3' to said region, and has, on its 5' side, a nucleotide sequence complementary to: a nucleotide sequence on the 5' side of the second chimeric oligonucleotide primer which is homologous to the nucleic acid as a template; a nucleotide sequence of the nucleic acid as a template corresponding to a region upstream of (i.e., 5' to) the 5' terminus of the portion homologous to the second chimeric oligonucleotide primer; or both.

In another embodiment of the present invention, the method for amplifying a nucleic acid comprises the steps of:

(a) preparing a reaction mixture by mixing a nucleic acid as a template, a deoxyribonucleotide triphosphate, a DNA polymerase having a reverse transcriptase activity, a DNA polymerase having a strand displacement activity, at least two chimeric oligonucleotide primers, at least one ladder-forming oligonucleotide primer and an RNase H; and
(b) incubating the reaction mixture for a sufficient time to generate a ladder-like amplification product under constant-temperature conditions under which specific annealing of the primer to the nucleic acid as a template, a reaction of synthesizing an extended strand and a strand displacement reaction by the DNA polymerase, as well as a reaction of cleaving an extended strand by the RNase H take place.

Each chimeric oligonucleotide primer contains a ribonucleotide as well as at least one selected from the group consisting of a deoxyribonucleotide and a nucleotide analog, and the ribonucleotide is positioned at the 3' terminus or on the 3'-terminal side of the primer.

The chimeric oligonucleotide primers comprise at least a first chimeric oligonucleotide primer which is complementary to a nucleotide sequence of the nucleic acid as a template and a second chimeric oligonucleotide primer which is homologous to a nucleotide sequence of the nucleic acid as a template.

The ladder-forming oligonucleotide primer is complementary to a region of the nucleic acid as a template that is complementary to the first chimeric oligonucleotide primer and a nucleotide sequence 3' to said region, and has, on its 5' side, a nucleotide sequence complementary to: a nucleotide sequence on the 5' side of the second chimeric oligonucleotide primer which is homologous to the nucleic acid as a template; a nucleotide sequence of the nucleic acid as a template corresponding to a region upstream of (i.e., 5' to) the 5' terminus of the portion homologous to the second chimeric oligonucleotide primer; or both.

In a further embodiment of the present invention, the method for amplifying a nucleic acid comprises the steps of:

(a) preparing a reaction mixture by mixing a nucleic acid as a template, a deoxyribonucleotide triphosphate, a DNA polymerase having a strand displacement activity, at least two chimeric oligonucleotide primers, at least one ladder-forming oligonucleotide primer and an RNase H; and
(b) incubating the reaction mixture for a sufficient time to generate a ladder-like amplification product under constant-temperature conditions under which specific annealing of the primer to the nucleic acid as a template, a reaction of synthesizing an extended strand and a strand displacement reaction by the DNA polymerase, as well as a reaction of cleaving an extended strand by the RNase H take place.

Each chimeric oligonucleotide primer contains a ribonucleotide as well as at least one selected from the group consisting of a deoxyribonucleotide and a nucleotide analog, and the ribonucleotide is positioned at the 3' terminus or on the 3'-terminal side of the primer.

The chimeric oligonucleotide primers comprise at least a first chimeric oligonucleotide primer which is complementary to a nucleotide sequence of the nucleic acid as a template and a second chimeric oligonucleotide primer which is homologous to a nucleotide sequence of the nucleic acid as a template.

The ladder-forming oligonucleotide primer is complementary to a region of the nucleic acid as a template that is complementary to the first chimeric oligonucleotide primer and a nucleotide sequence 3' to said region, and has, on its 5' side, a nucleotide sequence complementary to: a nucleotide sequence on the 5' side of the second chimeric oligonucleotide primer which is homologous to the nucleic acid as a template; a nucleotide sequence of the nucleic acid as a template corresponding to a region upstream of (i.e., 5' to) the 5' terminus of the portion homologous to the second chimeric oligonucleotide primer; or both.

In the above-mentioned embodiments, if the nucleic acid as a template is single-stranded, the first chimeric oligonucleotide primer has a nucleotide sequence complementary to the strand; the second chimeric oligonucleotide primer has a nucleotide sequence complementary to a strand complementary to the strand, that is, a nucleotide sequence homologous to the strand; and the ladder-forming chimeric oligonucleotide primer has a sequence nucleotide complementary to the strand and, on its 5' side, a nucleotide sequence complementary to: a nucleotide sequence on the 5' side of the second chimeric oligonucleotide primer which is homologous to the strand; a nucleotide sequence corresponding to a region upstream of the 5' terminus of the second chimeric oligonucleotide primer; or both. It is needless to say that in cases where the nucleic acid as a template is double-stranded the primers can be designed for one of the strands in a similar manner.

In any one of the above-mentioned embodiments, the ladder-forming oligonucleotide primer may be replaced by a chimeric oligonucleotide primer. In this case, the chimeric oligonucleotide primer can supplement the function of the ladder-forming oligonucleotide primer. Thus, an oligonucleotide primer and/or a chimeric oligonucleotide primer may be preferably used as long as it can contribute to ladder formation.

Although it is not intended to limit the present invention, for example, the first chimeric oligonucleotide primer can be used as the ladder-forming oligonucleotide primer. In this case, the first chimeric oligonucleotide primer has, on its 5' side, a nucleotide sequence complementary to: a nucleotide sequence on the 5' side of the second chimeric oligonucleotide primer which is homologous to the nucleic acid as a template; a nucleotide sequence corresponding to a region upstream of the 5' terminus of the second chimeric oligonucleotide primer; or both.

dNTPs (a mixture of dATP, dCTP, dGTP and dTTP) which are used for the PCR method or the like can be preferably used as nucleotide triphosphates that serve as substrates in the extension reaction in the method. In addition, dUTP may be used as a substrate. The dNTPs may contain an analog of dNTP (deoxyribonucleotide triphosphate) such as 7-deaza-dGTP, or a nucleotide triphosphate such as dITP as long as it can serve as a substrate for the DNA polymerase to be used. A derivative of a dNTP or a dNTP analog may be used. A derivative having a functional group such as a dUTP having an amino group may be contained. A chimeric oligonucleotide primer is used in the method. The primer can be prepared, for example, using a DNA synthesizer according to a conventional synthesis method.

The nucleic acid (DNA or RNA) used as a template in the method of the present invention may be prepared or isolated from any sample that may contain the nucleic acid. Alternatively, the sample may be used directly in the nucleic acid amplification reaction of the present invention. Examples of the samples that may contain the nucleic acid include, but are not limited to, samples from organisms such as whole blood, serum, buffy coat, urine, feces, cerebrospinal fluid, seminal fluid, saliva, tissue (e.g., cancerous tissue or lymph node) and cell culture (e.g., mammalian cell culture or bacterial cell culture), samples that contain a nucleic acid such as a viroid, a virus, a bacterium, a fungus, a yeast, a plant and an animal, samples suspected to be contaminated or infected with a microorganism such as a virus or a bacterium (e.g., food or a biological formulation), and samples that may contain an organism such as soil and waste water. The sample may be a nucleic acid-containing preparation obtained by processing the above-mentioned sample according to a known method. For example, a cell destruction product or a sample obtained by fractionating the product, a nucleic acid in the sample, or a sample in which a specific nucleic acid molecule population (e.g., mRNA) is enriched can be used as such a preparation according to the present invention. Also, a nucleic acid such as a DNA or an RNA obtained by amplifying a nucleic acid contained in the sample according to a known method can be preferably used.

A nucleic acid-containing preparation can be prepared from the above-mentioned material by using, for example, lysis with a detergent, sonication, shaking/stirring using glass beads or a French press, although it is not intended to limit the present invention. In some cases, it is advantageous to further process the preparation to purify the nucleic acid (e.g., in case where an endogenous nuclease exists). In such cases, the nucleic acid is purified using a known means such as phenol extraction, chromatography, ion exchange, gel electrophoresis or density-gradient centrifugation.

If it is desired to amplify a nucleic acid having a sequence derived from an RNA, the method of the present invention may be conducted using, as a template, a cDNA synthesized by a reverse transcription reaction in which the RNA is used as a template. Any RNA for which one can make a primer to be used in a reverse transcription reaction can be applied to the method of the present invention, including total RNA in a sample, an RNA molecule population such as mRNA, tRNA or rRNA, and specific RNA molecular species.

Any enzyme that has an activity of synthesizing a cDNA using an RNA as a template can be used in the reverse transcription reaction without limitation. Examples thereof include reverse transcriptases originating from various sources such as avian myeloblastosis virus-derived reverse transcriptase (AMV RTase), Molony murine leukemia virus-derived reverse transcriptase (M-MLV RTase) and Rous-associated virus 2 reverse transcriptase (RAV-2 RTase). In addition, a DNA polymerase additionally having a reverse transcription activity can be used. An enzyme having a reverse transcription activity at a high temperature is preferable for the purpose of the present invention. For example, a DNA polymerase from a bacterium of the genus *Thermus* (e.g., Tth DNA polymerase), a DNA polymerase from a thermophilic bacterium of the genus *Bacillus* or the like can be used. Although it is not intended to limit the present invention, for example, DNA polymerases from thermophilic bacteria of the genus *Bacillus* such as a DNA polymerase from B. st (Bst DNA polymerase) and Bca DNA polymerase are preferable. For example, Bca DNA polymerase does not require a manganese ion for the reverse transcription reaction. Furthermore, it can synthesize a cDNA while suppressing secondary structure formation of a template RNA under high-temperature conditions. Both a naturally occurring enzyme and a variant of the enzyme having a reverse transcriptase activity can be used as long as they have the activity.

Although it is not intended to limit the present invention, an oligonucleotide of preferably about 6 nucleotides to about 50 nucleotides, more preferably about 10 nucleotides to about 40 nucleotides, still more preferably about 12 nucleotides to about 30 nucleotides can be used as chimeric oligonucleotide primer according to the method of the present invention. It is preferable that the nucleotide sequence of the chimeric oligonucleotide primer is complementary to the template nucleic acid such that it anneals to the nucleic acid as a template under the reaction conditions used. The primer contains a sequence recognized by an RNase H, which is used in a step as described below, at the 3' terminus or on the 3'-terminal side.

Although it is not intended to limit the present invention, for example, an oligonucleotide having a structure represented by the following general formula can be used as primer in the DNA synthesis method according to the present invention:

General formula: 5'-dNa-Nb-dNc-3'

(in the general formula, "dN" represents deoxyribonucleotide and/or nucleotide analog, and "N" represents unmodified ribonucleotide and/or modified ribonucleotide, wherein an analog of a nucleotide or a derivative of a nucleotide (a modified nucleotide) may be contained for each nucleotide as long as the function is not impaired, and some of the dNs in "dNa" may be replaced by Ns).

In the above-mentioned general formula, for example, "a" is an integer of 5 or more, preferably 6 or more, more preferably 8 or more; "b" is an integer of 1 or more, for example 1-15, preferably 1-10, more preferably 1-7, still more preferably 1-5; and "c" may be 0 or c may be an integer of 1 or more, preferably 0-5, more preferably 0-3, although it is not intended to limit the present invention.

According to the method of the present invention, a ladder-forming oligonucleotide primer may be used for obtaining a ladder-like amplification product. At least one selected from the group consisting of a deoxyribonucleotide, a ribonucleotides and a nucleotide analog can be preferably used for the primer.

It is preferable to use a ladder-forming oligonucleotide primer in the method of the present invention having, on its 5' side, an attached nucleotide sequence complementary to: a nucleotide sequence on the 5' side of the second chimeric oligonucleotide primer which is homologous to the nucleic acid as a template; a nucleotide sequence corresponding to a region upstream of the 5' terminus of the second chimeric oligonucleotide primer; or both.

If an RNA is used as template according to the present invention, the ladder-forming oligonucleotide primer may be used as primer for reverse transcription. There is no specific limitation as long as it anneals to the template RNA under the reaction conditions used. The primer may be a primer having a nucleotide sequence that is complementary to a specific template RNA (a specific primer), an oligo-dT (deoxythymine) primer and a primer having a random sequence (a random primer). In view of specific annealing, the length of the portion of the sequence complementary to a part of the nucleotide sequence of the template nucleic acid in the ladder-forming oligonucleotide primer is preferably 6 nucleotides or more, more preferably 9 nucleotides or more. In view of oligonucleotide synthesis, the length is preferably 100 nucleotides or less, more preferably 30 nucleotides or less. There is no specific limitation as long as it can be used for a reverse transcription reaction from an RNA.

There is no specific limitation concerning the ladder-forming oligonucleotide primer as long as it can contribute to extension of a DNA strand in a ladder-like pattern under the conditions used. The primer preferably has a nucleotide sequence complementary to: a nucleotide sequence on the 5' side of the chimeric oligonucleotide primer that is homologous to the nucleic acid as a template; a nucleotide sequence corresponding to a region upstream of the 5' terminus of the chimeric oligonucleotide primer; or both. Although it is not intended to limit the present invention, the length of the sequence complementary to the region upstream of the chimeric oligonucleotide primer is preferably about 3 to about 100 nucleotide, more preferably about 4 to about 50 nucleotides, still more preferably about 5 to about 20 nucleotides.

Although it is not intended to limit the present invention, the sequence complementary to a region upstream of the chimeric oligonucleotide primer is a sequence of 3 nucleotides or more selected from position +20 to position -100, preferably position +15 to position -80, more preferably position +10 to position -60, still more preferably position +5 to position -40. Examples of such nucleotide sequences include, but are not limited to, regions starting from position -1, position -11 or position -21.

There is no specific limitation concerning the portion to which the ladder-forming oligonucleotide primer anneals as long as the function can be exerted. The portion may be a portion overlapping entirely or by one or more nucleotide (s) with, adjacent to, or at a distance of 1 to 40 nucleotide(s) from the chimeric oligonucleotide primer.

Although it is not intended to limit the present invention, the GC content of the ladder-forming oligonucleotide primer is preferably 40% or more.

Furthermore, the ladder-forming oligonucleotide primer used in the method of the present invention may contain a nucleotide analog or other substances. That is, one or more nucleotide analog(s) can be contained in the ladder-forming oligonucleotide primer used according to the present invention as long as the function of the primer for effecting a polymerase extension reaction from the 3' terminus by the action of a DNA polymerase is not abolished. Plural types of the nucleotide analogs can be used in combination. Examples of the nucleotide analogs that can be used include, but are not limited to, deoxyinosine nucleotide, deoxyuracil nucleotide, a nucleotide analog having a modified base such as 7-deazaguanine, a nucleotide analog having a ribose derivative and the like. Furthermore, the chimeric oligonucleotide primer used according to the present invention may contain a deoxynucleotide, a ribonucleotide or a nucleotide analog having one of various modifications such as addition of a labeled compound or addition of a functional group for immobilization as long as the functions as described above are retained.

Incorporation of a nucleotide analog into a primer is effective for suppressing formation of a high-order structure of the primer itself and stabilizing a form annealed to a template.

The chimeric oligonucleotide primer and the ladder-forming oligonucleotide primer used in the method of the present invention can be synthesized to have arbitrary nucleotide sequences using, for example, Applied Biosystems Inc. (ABI) 394 DNA synthesizer according to the phosphoramidite method. Alternatively, any methods including the phosphate triester method, the H-phosphonate method and the thiophosphonate method may be used for the synthesis.

In another embodiment of the present invention, it is possible to obtain a ladder-like amplification product utilizing a nucleotide sequence inherently present in the nucleic acid as a template. In this case, it is possible to carry out ladder formation utilizing the nucleotide sequence in the nucleic acid as a template. Although it is not intended to limit the present invention, for example, if a nucleotide sequence on the 5' side of or upstream of one of the chimeric oligonucleotide primers is complementary to a nucleotide sequence on the 5' side of or upstream of the other chimeric oligonucleotide primer, a ladder-like amplification product can be efficiently obtained by designing the chimeric oligonucleotide primers for such positions. Detection sensitivity and detection velocity are increased by carrying out ladder formation.

The complementary nucleotide sequences in the nucleic acid as a template on the 5' sides of or upstream of the chimeric oligonucleotide primers can be arbitrarily selected depending on the GC contents, the adjacent sequences, the distances from the primers, the lengths to be amplified, the reaction conditions and the like. Although it is not intended to limit the present invention, for example, they are preferably selected from complementary nucleotide sequences of 3 nucleotides or more, preferably 6 nucleotides or more. Furthermore, although it is not intended to limit the present invention, the sequence is for example a region from +20 to -100, preferably from +15 to -80, more preferably from +10 to -60, still more preferably from +5 to -40, defining the 5' terminus of the chimeric oligonucleotide primer as position 0. The complementary nucleotide sequences in the nucleic acid as a template on the 5' sides of or upstream of the chimeric oligonucleotide primers can be arbitrarily selected depending on the GC contents, the adjacent sequences, the distances from the primers, the lengths to be amplified, the reaction conditions and the like. Although it is not intended to limit the present invention, the complementarities are preferably 70% or more.

Although it is not intended to limit the present invention, the ladder-forming oligonucleotide primer used in the method of the present invention is described referring, as examples, to the ladder-forming oligonucleotide primers used in the Examples below.

For example, in A12-205R (SEQ ID NO:3), the nucleotides at positions 1 to 12 constitute a nucleotide sequence complementary to a region 1 to 12 nucleotides upstream of the 5' terminus (-1 to -12) of the second chimeric oligonucleotide primer 134FN3(18) (SEQ ID NO:7), and the nucleotides at positions 13 to 30 constitute a sequence complementary to the region complementary to the first chimeric oligonucleotide primer 205RN3(18) (SEQ ID NO:2). In other words, the nucleotides at positions 13 to 30 constitute a sequence homologous to the first chimeric oligonucleotide primer.

For example, in A12-215R (SEQ ID NO:5), the nucleotides at positions 1 to 12 constitute a nucleotide sequence complementary to a region 1 to 12 nucleotides upstream of the 5' terminus (-1 to -12) of the second chimeric oligonucleotide primer 134FN3(18) (SEQ ID NO:7), and the nucleotides at positions 13 to 30 constitute a region complementary to an 8-nucleotide sequence on the 5' side of the first chimeric oligonucleotide primer 205RN3(18) (SEQ ID NO:2) and a sequence complementary to a 10-nucleotide sequence 3' to the region complementary to the first chimeric oligonucleotide primer. In other words, the nucleotides at positions 13 to 30 constitute a sequence homologous to a region starting from the 10th upstream nucleotide to the 8-nucleotide sequence on the 5' side of the first chimeric oligonucleotide primer. The nucleotides at positions 23 to 30 of A12-215R overlap with the nucleotides at positions 1 to 8 of 205RN3(18).

For example, in A12-223R (SEQ ID NO:5), the nucleotides at positions 1 to 12 constitute a nucleotide sequence complementary to a region 1 to 12 nucleotides upstream of the 5' terminus (-1 to -12) of the second chimeric oligonucleotide primer 134FN3(18) (SEQ ID NO:7), and the nucleotides at positions 13 to 30 constitute a sequence complementary to an 18-nucleotide sequence 3' to the region complementary to the first chimeric oligonucleotide primer 205RN3(18) (SEQ ID NO:2). In other words, the nucleotides at positions 13 to 30 constitute a sequence homologous to a sequence from the 18th upstream nucleotide to the 1st upstream nucleotide of the first chimeric oligonucleotide primer. A12-223R is adjacent to 205RN3(18).

For example, in A12(-10)-215R (SEQ ID NO:8), the nucleotides at positions 1 to 12 constitute a nucleotide sequence complementary to a region 11 to 22 nucleotides upstream of the 5' terminus (-11 to -22) of the second chimeric oligonucleotide primer 134FN3(18) (SEQ ID NO:7), and the nucleotides at positions 13 to 30 constitute a region complementary to an 8-nucleotide sequence on the 5' side of the first chimeric oligonucleotide primer 205RN3(18) (SEQ ID NO:2) and a sequence complementary to a 10-nucleotide sequence 3' to the region complementary to the first chimeric oligonucleotide primer. In other words, the nucleotides at positions 13 to 30 constitute a sequence homologous to a region starting from the 10th upstream nucleotide to the 8-nucleotide sequence on the 5' side of the first chimeric oligonucleotide primer. The nucleotides at positions 23 to 30 of A12(-10)-215R overlap with the nucleotides at positions 1 to 8 of 205RN3(18).

For example, in A12(-20)-215R (SEQ ID NO:9), the nucleotides at positions 1 to 12 constitute a nucleotide sequence complementary to a region 21 to 32 nucleotides upstream of the 5' terminus (-21 to -32) of the second chimeric oligonucleotide primer 134FN3(18) (SEQ ID NO:7), and the nucleotides at positions 13 to 30 constitute a region complementary to an 8-nucleotide sequence on the 5' side of the first chimeric oligonucleotide primer 205RN3(18) (SEQ ID NO:2) and a sequence complementary to a 10-nucleotide sequence 3' to the region complementary to the first chimeric oligonucleotide primer. In other words, the nucleotides at positions 13 to 30 constitute a sequence homologous to a region starting from the 10th upstream nucleotide to the 8-nucleotide sequence on the 5' side of the first chimeric oligonucleotide primer. The nucleotides at positions 23 to 30 of A12(-20)-215R overlap with the nucleotides at positions 1 to 8 of 205RN3(18).

For example, in A12(6)-215R (SEQ ID NO:10), the nucleotides at positions 1 to 12 constitute a nucleotide sequence complementary to a 6-nucleotide sequence on the 5' side (+5 to 0) and a region 1 to 6 nucleotides upstream of the 5' terminus (-1 to -6) of the second chimeric oligonucleotide primer 134FN3(18) (SEQ ID NO:7), and the nucleotides at positions 13 to 30 constitute a region complementary to an 8-nucleotide sequence on the 5' side of the first chimeric oligonucleotide primer 205RN3(18) (SEQ ID NO:2) and a sequence complementary to a 10-nucleotide sequence 3' to the region complementary to the first chimeric oligonucleotide primer. In other words, the nucleotides at positions 13 to 30 constitute a sequence homologous to a region starting from the 10th upstream nucleotide to the 8-nucleotide sequence on the 5' side of the first chimeric oligonucleotide primer. The nucleotides at positions 23 to 30 of A12(6)-215R overlap with the nucleotides at positions 1 to 8 of 205RN3(18).

For example, in A12(12)-215R (SEQ ID NO:11), the nucleotides at positions 1 to 12 constitute a nucleotide sequence complementary to a 12-nucleotide sequence on the 5' side (+11 to 0) of the second chimeric oligonucleotide primer 134FN3(18) (SEQ ID NO:7), and the nucleotides at positions 13 to 30 constitute a region complementary to an 8-nucleotide sequence on the 5' side of the first chimeric oligonucleotide primer 205RN3(18) (SEQ ID NO:2) and a sequence complementary to a 10-nucleotide sequence 3' to the region complementary to the first chimeric oligonucleotide primer. In other words, the nucleotides at positions 13 to 30 constitute a sequence homologous to a region starting from the 10th upstream nucleotide to the 8-nucleotide sequence on the 5' side of the first chimeric oligonucleotide primer. The nucleotides at positions 23 to 30 of A12(12)-215R overlap with the nucleotides at positions 1 to 8 of 205RN3(18).

For example, in A6 (-10) -215R (SEQ ID NO:14), the nucleotides at positions 1 to 6 constitute a nucleotide sequence complementary to a region 11 to 16 nucleotides upstream of the 5' terminus (-11 to -16) of the second chimeric oligonucleotide primer 134FN3(18) (SEQ ID NO:7), and the nucleotides at positions 7 to 24 constitute a region complementary to an 8-nucleotide sequence on the 5' side of the first chimeric oligonucleotide primer 205RN3(18) (SEQ ID NO:2) and a sequence complementary to a 10-nucleotide sequence 3' to the region complementary to the first chimeric oligonucleotide primer. In other words, the nucleotides at positions 7 to 24 constitute a sequence homologous to a region starting from the 10th upstream nucleotide to the 8-nucleotide sequence on the 5' side of the first chimeric oligonucleotide primer. The nucleotides at positions 17 to 24 of A6(-10)-215R overlap with the nucleotides at positions 1 to 8 of 205RN3(18).

For example, in A9(-10)-215R (SEQ ID NO:15), the nucleotides at positions 1 to 9 constitute a nucleotide sequence complementary to a region 11 to 19 nucleotides upstream of the 5' terminus (-11 to -19) of the second chimeric oligonucleotide primer 134FN3(18) (SEQ ID NO:7), and the nucleotides at positions 10 to 27 constitute a region complementary to an 8-nucleotide sequence on the 5' side of the first chimeric oligonucleotide primer 205RN3(18) (SEQ ID NO:2) and a sequence complementary to a 10-nucleotide sequence 3' to the region complementary to the first chimeric oligonucleotide primer. In other words, the nucleotides at positions 10 to 27 constitute a sequence homologous to a region starting from the 10th upstream nucleotide to the 8-nucleotide sequence on the 5' side of the first chimeric oligonucleotide primer. The nucleotides at positions 20 to 27 of A9(-10)-215R overlap with the nucleotides at positions 1 to 8 of 205RN3(18).

For example, in (A12)241RN3 (SEQ ID NO:19), the nucleotides at positions 1 to 7 constitute a nucleotide sequence complementary to a region 1 to 7 nucleotides upstream of the 5' terminus (-1 to -7) of the second chimeric oligonucleotide primer 160FN3 (SEQ ID NO:17), and the nucleotides at positions 8 to 21 constitute a sequence complementary to the region complementary to the first chimeric oligonucleotide primer 241RN3 (SEQ ID NO:18). In other words, the nucleotides at positions 8 to 21 constitute a sequence homologous to the first chimeric oligonucleotide primer.

For example, in ALDH2-TH1 (SEQ ID NO:25), the nucleotides at positions 1 to 12 constitute a nucleotide sequence complementary to a region 1 to 12 nucleotides upstream of the 5' terminus (-1 to -12) of the second chimeric oligonucleotide primer ICAN-ALDH2-F (SEQ ID NO:21), and the nucleotides at positions 13 to 29 constitute a region of the nucleic acid as a template complementary to a 15-nucleotide sequence on the 5' side of the first chimeric oligonucleotide primer ICAN-ALDH2-R (SEQ ID NO:22) and a sequence complementary to a 2-nucleotide sequence 3' to the region complementary to the first chimeric oligonucleotide primer. In other words, they constitute a sequence homologous to a region starting from the 2nd upstream nucleotide to the 15-nucleotide sequence on the 5' side of the first chimeric oligonucleotide primer. The nucleotides at positions 2 to 16 of ICAN-ALDH2-R overlap with the nucleotides at positions 15 to 29 of ALDH2-TH1.

For example, in ALDH2-TH2 (SEQ ID NO:26), the nucleotides at positions 1 to 12 constitute a nucleotide sequence complementary to a region 1 to 12 nucleotides upstream of the 5' terminus (-1 to -12) of the second chimeric oligonucleotide primer ICAN-ALDH2-F (SEQ ID NO:21), and the nucleotides at positions 13 to 29 constitute a region complementary to a 6-nucleotide sequence on the 5' side of the first chimeric oligonucleotide primer ICAN-ALDH2-R (SEQ ID NO:22) and a sequence complementary to a 10-nucleotide sequence 3' to the region complementary to the first chimeric oligonucleotide primer. In other words, the nucleotides at positions 13 to 29 constitute a sequence homologous to a region starting from the 10th upstream nucleotide to the 6-nucleotide sequence on the 5' side of the first chimeric oligonucleotide primer. The nucleotides at positions 2 to 7 of ICAN-ALDH2-R overlap with the nucleotides at positions 23 to 28 of ALDH2-TH2.

For example, in ALDH2-TH3 (SEQ ID NO:27), the nucleotides at positions 1 to 12 constitute a nucleotide sequence complementary to a region 1 to 12 nucleotides upstream of the 5' terminus (-1 to -12) of the second chimeric oligonucleotide primer ICAN-ALDH2-F (SEQ ID NO:21), and the nucleotides at positions 13 to 28 constitute a sequence complementary to a nucleotide sequence 2 to 17 nucleotides 3' to the sequence complementary to the first chimeric oligonucleotide primer ICAN-ALDH2-R (SEQ ID NO:22). In other words, the nucleotides at positions 13 to 28 constitute a sequence homologous to a sequence 2 to 17 nucleotides upstream of the first chimeric oligonucleotide primer.

For example, in R2(-13)A12-1 (SEQ ID NO:35), the nucleotides at positions 1 to 12 constitute a nucleotide sequence complementary to a region 1 to 12 nucleotides upstream of the 5' terminus (-1 to -12) of the second chimeric oligonucleotide primer F2 (SEQ ID NO:31), and the nucleotides at positions 13 to 29 constitute a sequence complementary to a nucleotide sequence 13 to 29 nucleotides 3' to the sequence complementary to the first chimeric oligonucleotide primer R2 (SEQ ID NO:32). In other words, the nucleotides at positions 13 to 29 constitute a sequence homologous to a sequence 13 to 29 nucleotides upstream of the first chimeric oligonucleotide primer.

For example, in R2(-13)A12-2 (SEQ ID NO:36), the nucleotides at positions 1 to 12 constitute a nucleotide sequence complementary to a region 13 to 24 nucleotides upstream of the 5' terminus (-13 to -24) of the second chimeric oligonucleotide primer F2 (SEQ ID NO:31), and the nucleotides at positions 13 to 29 constitute a sequence complementary to a nucleotide sequence 13 to 29 nucleotides 3' to the sequence complementary to the first chimeric oligonucleotide primer R2 (SEQ ID NO:32). In other words, the nucleotides at positions 13 to 29 constitute a sequence homologous to a sequence 13 to 29 nucleotides upstream of the first chimeric oligonucleotide primer.

For example, in A6-215R (SEQ ID NO:47), the nucleotides at positions 1 to 6 constitute a nucleotide sequence complementary to a region 1 to 6 nucleotides upstream of the 5' terminus (-1 to -6) of the second chimeric oligonucleotide primer B134FN3 (SEQ ID NO:12), and the nucleotides at positions 7 to 24 constitute a region complementary to an 8-nucleotide sequence on the 5' side of the first chimeric oligonucleotide primer 205RN3(16) (SEQ ID NO:13) and a sequence complementary to a 10-nucleotide sequence 3' to the region complementary to the first chimeric oligonucleotide primer. In other words, the nucleotides at positions 7 to 24 constitute a sequence homologous to a region starting from the 10th upstream nucleotide to the 8-nucleotide sequence on the 5' side of the first chimeric oligonucleotide primer. The nucleotides at positions 1 to 8 of 205RN3(16) overlap with the nucleotides at positions 17 to 24 of A6-215R.

For example, in A9-215R (SEQ ID NO:48), the nucleotides at positions 1 to 9 constitute a nucleotide sequence complementary to a region 1 to 9 nucleotides upstream of the 5' terminus (-1 to -9) of the second chimeric oligonucleotide primer B134FN3 (SEQ ID NO:12), and the nucleotides at positions 10 to 27 constitute a region complementary to an 8-nucleotide sequence on the 5' side of the first chimeric oligonucleotide primer 205RN3(16) (SEQ ID NO:13) and a sequence complementary to a 10-nucleotide sequence 3' to the region complementary to the first chimeric oligonucleotide primer. In other words, the nucleotides at positions 7 to 24 constitute a sequence homologous to a region starting from the 10th upstream nucleotide to the 8-nucleotide sequence on the 5' side of the first chimeric oligonucleotide primer. The nucleotides at positions 1 to 8 of 205RN3(16) overlap with the nucleotides at positions 20 to 27 of A6-215R.

Positional relationships among the first chimeric oligonucleotide primer, the second chimeric oligonucleotide primer and the ladder-forming oligonucleotide primers used in Example 2-(1)-(A) are illustrated in Figure 1.

Any RNase H can be used in the method of the present invention, including mesophilic and heat-resistant ones. For example, RNase HII from *Thermococcus litralis* (hereinafter referred to as Tli RNase HII) prepared according to the method as described in Example 8 of WO 02/22831 or RNase HII from *Archaeoglobus fulgidus* (hereinafter referred to as Afu RNase HII) prepared according to the method as described in Example 7 of WO 02/22831 can be used in the method of the present invention as described in the Examples below. Examples of heat-resistant RNase Hs that can be preferably used include, but are not limited to, a commercially available RNase H, Hybridase™ Thermostable RNase H (Epicentre Biotechnologies) as well as an RNase H from a thermophilic bacterium of the genus *Bacillus*, a bacterium of the genus *Thermus*, a bacterium of the genus *Pyrococcus*, a bacterium of the genus *Thermotoga*, a bacterium of the genus *Archaeoglobus* or the like. Furthermore, both, naturally occurring RNase Hs and variants can be used.

Any DNA polymerases having strand displacement activity can be used according to the present invention. Examples thereof include variants of DNA polymerases lacking their 5'→3' exonuclease activities derived from thermophilic bacteria of the genus *Bacillus* such as *Bacillus caldotenax* (hereinafter referred to as B. ca) and *Bacillus stearothermophilus* (hereinafter referred to as B. st), as well as the large fragment (Klenow fragment) of DNA polymerase I from *Escherichia coli* (*E. coli*). Both mesophilic and heat-resistant DNA polymerases can be preferably used according to the present invention.

B. ca is a thermophilic bacterium having an optimal growth temperature of about 70°C. Bca DNA polymerase from this bacterium is known to have DNA-dependent DNA polymerase activity, RNA-dependent DNA polymerase activity (reverse transcription activity), 5'→3' exonuclease activity and 3'→5' exonuclease activity. The enzyme may be either an enzyme purified from its original source or a recombinant protein produced by using genetic engineering techniques. The enzyme may be subjected to modification such as substitution, deletion, addition or insertion by using genetic engineering techniques or other means. Examples of such enzymes include BcaBEST DNA polymerase (Takara Bio), which is Bca DNA polymerase lacking its 5'→3' exonuclease activity. This enzyme can be prepared according to the method as described in Japanese Patent No. 2978001 from the microorganism disclosed therein.

It is known that a certain DNA polymerase has endonuclease activity such as RNase H activity under specific conditions. Such a DNA polymerase can be used in the method of the present invention. In one aspect, the DNA polymerase may be used under conditions under which RNase H activity is exhibited, e.g., in the presence of $Mn^{2+}$. In this case, the method of the present invention can be conducted without the addition of an RNase H. Thus, the Bca DNA polymerase can exhibit RNase activity in a buffer containing $Mn^{2+}$. The above-mentioned aspect is not limited to the use of the Bca DNA polymerase. A DNA polymerase known to additionally have RNase H activity such as Tth DNA polymerase from *Thermus thermophilus* can be used according to the present invention.

A ladder-forming oligonucleotide primer may be used in the method of the present invention; the first chimeric oligonucleotide primer may also function as ladder-forming oligonucleotide primer; or a ladder-like amplification product may be obtained utilizing a complementary nucleotide sequence in the nucleic acid as a template. In either case, it is possible to stabilize the reaction by conducting the reaction in the presence of a random primer to efficiently obtain the amplification product of interest. Although there is no specific limitation concerning the random primer, for example, a random primer of 6 to 9 nucleotides in length can be preferably used.

(2) Composition used for the method of the present invention

A composition used for the method for amplifying a nucleic acid of the present invention or the method for detecting a nucleic acid of the present invention as described above may contain, for example, a ladder-forming oligonucleotide primer and/or a chimeric oligonucleotide primer, an RNase H and a DNA polymerase as described in (1) above. The composition may further contain a buffering component, a magnesium salt, dNTPs and the like as components for conducting an amplification reaction. Furthermore, it may contain a modified deoxyribonucleotide or a deoxynucleotide triphosphate analog. Additionally, a buffering component and other additives can be used.

In a particularly preferable aspect, the composition may contain suitable amounts of the various components as listed above for the nucleic acid amplification method of the present invention. An amplification reaction can be conducted only by adding an appropriate template, a chimeric oligonucleotide primer and/or a ladder-forming oligonucleotide to the composition. If the subject to be amplified is known beforehand, the composition preferably contains a chimeric oligonucleotide primer suitable for the amplification of the subject.

(3) Kit used for the method of the present invention

In one embodiment, a kit used for the method for amplifying a nucleic acid of present invention as described above is in a packed form and contains instructions regarding the use of a DNA polymerase, an RNase H, a chimeric oligonucleotide primer and/or a ladder-forming oligonucleotide primer in a strand displacement reaction. Also, a kit that contains a DNA polymerase having strand displacement activity, an RNase H, and a buffer for a strand displacement reaction is preferably used for the method of the present invention. Alternatively, a commercially available DNA polymerase having strand displacement activity and/or RNase H may be selected and used according to the instructions. Additionally, the kit may contain a reagent for a reverse transcription reaction which is used if an RNA is to be used as template. The DNA polymerase can be selected from the DMA polymerases to be used according to the present invention as described in (1) above. The RNase H can be selected from the RNase Hs as described in (1) above.

"Instructions" are printed matters describing a method of using the kit, e.g., a method of preparing a reagent solution for a strand displacement reaction, recommended reaction conditions and the like. The instructions include an instruction manual in the form of a pamphlet or a leaflet, a label stuck to the kit, and description on the surface of the package containing the kit. The instructions also include information disclosed or provided through electronic media such as the Internet.

The kit may further contain a reaction buffer containing Bicine, Tricine, HEPES, phosphate or tris-hydrochloride as a buffering component and an annealing solution. Additionally, it may contain a DNA polymerase having strand displacement activity and an RNase H. Furthermore, the kit may contain a modified deoxyribonucleotide or a deoxynucleotide triphosphate analog.

The kit used in the method for detecting a target nucleic acid may further contain a chimeric oligonucleotide primer and/or a ladder-forming oligonucleotide primer suitable for amplification of a target nucleic acid, and a reagent for detecting the amplified target nucleic acid (e.g., a probe) in addition to the instructions and the reagents for amplification reaction as described above.

(4) The method for detecting a target nucleic acid of the present invention

It is possible to detect a target nucleic acid in a sample using the method for amplifying a nucleic acid of the present invention. The detection method comprises the steps of:

(a) amplifying a target nucleic acid according to the method for amplifying a nucleic acid of the present invention as described in (1) above; and
(b) detecting the target nucleic acid amplified in the above step.

If an RNA is to be used as template in step (a) above, the reverse transcription reaction and the nucleic acid amplification reaction may be conducted in one or two step(s). Although it is not intended to limit the present invention, for example, a combination of AMV RTase, M-MLV RTase or RAV-2 RTase and BcaBEST DNA polymerase can be preferably used as a combination of a reverse transcriptase and a strand displacement-type DNA polymerase. Alternatively, a DNA polymerase having reverse transcription activity and strand displacement activity may be used. For example, BcaBEST DNA polymerase can be preferably used.

Information about a specific nucleotide in a gene such as a point mutation or a single nucleotide polymorphism (SNP) can be obtained using the method for detecting a target nucleic acid of the present invention. In this embodiment, the 3'-terminal portion of the chimeric oligonucleotide primer to be used may be placed in the vicinity of the specific nucleotide in the target nucleotide sequence to be distinguished.

In addition, a target nucleic acid can be detected with higher sensitivity even from a trace amount of a nucleic acid sample according to the detection method of the present invention by using a reaction buffer containing Bicine, Tricine, HEPES, phosphate or tris as a buffering component and an annealing solution containing spermidine or propylenediamine. In this case, the RNase H and the DNA polymerase to be used are not limited to specific ones. For example, a combination of RNase HII from a bacterium of the genus *Thermococcus* and BcaBEST DNA polymerase is preferable. It is considered that the preferable unit numbers of the RNase H and the DNA polymerase may vary depending on the types of the enzymes. In such a case, the composition of the buffer and the amounts of the enzymes to be added may be adjusted using the increase in detection sensitivity or the amount of amplification product as an index.

According to the detection method of the present invention, dUTP may be incorporated as a substrate during amplification of a target nucleic acid. Thus, if dUTP is used as a substrate, it is possible to prevent false positives due to carry-over contamination of amplification products by degrading amplification products utilizing uracil N-glycosidase (UNG).

A known method for detecting a nucleic acid can be used for step (b). For example, detection of a reaction product having a specific size by electrophoresis, real-time detection using Smart Cycler (Takara Bio), Rotor gene (Corbett Research) or the like, or detection by hybridization with a probe can be used. Furthermore, a detection method in which magnetic beads are used in combination can be preferably used. The reaction mixture may be made turbid by converting pyrophosphoric acid generated during the step of amplification of the target nucleic acid into an insoluble substance such as a magnesium salt, and then the turbidity may be measured.

A fluorescent substance such as ethidium bromide is usually used for the detection by electrophoresis. The hybridization with a probe may be combined with the detection by electrophoresis. The probe may be labeled with a radioisotope or with a non-radioactive substance such as biotin or a fluorescent substance. If real-time detection is to be carried out, a coloring reagent such as SYBR Green (Takara Bio) can be preferably used. Additionally, use of a labeled nucleotide in step (a) may facilitate the detection of amplification product into which the labeled nucleotide is incorporated, or may enhance the signal for detection utilizing the label. A fluorescence polarization method, fluorescence energy transition or the like can also be utilized for the detection. The target nucleic acid can be detected automatically or quantified by constructing a suitable detection system. In addition, detection with the naked eye using a hybrid chromatography method can be preferably used.

A ribonucleotide (RNA) probe, or a chimeric oligonucleotide probe composed of ribonucleotides and deoxyribonucleotides, labeled with two or more fluorescent substances positioned at a distance that results in a quenching state can be used in the detection method of the present invention. The probe does not emit fluorescence. When it is annealed to a DNA amplified from a target nucleic acid that is complementary to the probe, RNase H digests the probe. The distance between the fluorescent substances on the probe then increases, resulting in the emission of fluorescence. Thus, the emission reveals the presence of the target nucleic acid. If RNase H is used in the method for amplifying a nucleic acid of the present invention, a target nucleic acid can be detected only by adding the probe to the reaction mixture. For example, a combination of the fluorescent substance ROX (Applied Biosystems) or FAM (Applied Biosystems) and the quenching substance Eclipse (Epoch Biosciences) can be preferably used for labeling the probe.

The probe used according to the present invention is not limited to a specific one as long as it can hybridize to a target nucleic acid amplified according to the nucleic acid amplification method of the present invention under normal hybridization conditions. In view of specific detection of amplification product, a probe that hybridizes under conditions, for example, known to those skilled in the art as being stringent is preferable. The stringent hybridization conditions are described in, for example, T. Maniatis et al. (eds.), Molecular Cloning: A Laboratory Manual 2nd ed., 1989, Cold Spring Harbor Laboratory. Specifically, the stringent conditions are exemplified by the following: incubation at a temperature about 25°C lower than the Tm value of the probe to be used for 4 hours to overnight in 6 x SSC (1 x SSC: 0.15 M NaCl, 0.015 M sodium citrate, pH 7.0) containing 0.5% SDS, 5 x Denhardt's (0.1% bovine serum albumin (BSA), 0.1% polyvinylpyrrolidone, 0.1% Ficoll 400) and 100 $\mu$g/ml salmon sperm DNA. A probe having a label as described above may be used as the probe for facilitating the detection of the target nucleic acid.

A ladder-like amplification product in which amplified regions are connected to each other is positively generated according to the method for detecting a target nucleic acid of the present invention. In the ladder-like amplification product, plural amplified regions are polymerized through an arbitrary nucleotide sequence in the same direction. The amplification product is observed as a ladder-like band upon analysis of the amplification product by electrophoresis. The generation of the ladder-like amplification product can be adjusted depending on the region to be amplified, the size of the region, the adjacent region, the nucleotide sequence of the chimeric oligonucleotide primer and/or the ladder-forming oligonucleotide primer to be used, the reaction conditions or the like.

According to the detection method of the present invention, the ladder-forming oligonucleotide primer and/or the chimeric oligonucleotide primer can be used to generate a polymer in which amplified regions are connected to each other. In the ladder-like amplification product, plural amplified regions are connected to each other in the same direction through the ladder-forming oligonucleotide primer and a sequence complementary to a region on the 5' side of or upstream of a second chimeric oligonucleotide primer. Furthermore, according to the method of the present invention, if a nucleotide sequence of a template nucleic acid on the 5' side of or upstream of one of chimeric oligonucleotide primers is complementary to a nucleotide sequence on the 5' side of or upstream of the other chimeric oligonucleotide primer, the target nucleic acid can be detected with high sensitivity by designing the chimeric oligonucleotide primers for the portions.

The ladder-like amplification product contains plural amplified regions. Therefore, it can be hybridized with many copies of an appropriate probe, for example. One that hybridizes to the nucleotide sequence of the target nucleic acid and/or the intervening sequence existing between the target nucleic acid sequences in the ladder-like amplification product can be preferably used as such a probe. Since an intense signal is consequently emitted, the present invention is useful if a nucleic acid containing an amplified region is to be detected. It is possible to obtain an amplified region or a part thereof as a monomer from a ladder-like amplification product by using restriction enzyme digestion or the like in combination.

The isothermal nucleic acid amplification method of the present invention does not require the use of equipment such as a thermal cycler. Since reagents such as dNTPs used for PCR and the like can be applied to the method, the running cost can be reduced as compared with a conventional method. Therefore, the method of the present invention can be preferably used, for example, in the field of genetic tests in which the detection is routinely conducted. The method of the present invention provides a greater amount of an amplification product in a shorter time than PCR. Therefore, the method of the present invention can be utilized as a convenient, rapid and sensitive gene detection method.

Examples

The following examples further illustrate the present invention in detail but are not to be construed to limit the scope thereof.

Referential Example 1

The unit number of the heat-resistant RNase H in the Examples was calculated as follows.
1 mg of poly(rA) or poly(dT) (both from Amersham Biotech) was dissolved in 1 ml of 40 mM tris-HCl (pH 7.7) containing 1 mM EDTA to prepare a poly (rA) solution and a poly(dT) solution.
The poly(rA) solution (to a final concentration of 30 $\mu$g/ml) and the poly(dT) solution (to a final concentration of 20 $\mu$g/ml) were then added to 20 mM HEPES-KOH (pH 7.8), 100 mM potassium acetate, 1% dimethyl sulfoxide and 0.01% BSA. The mixture was incubated at 37°C for 10 minutes and then cooled to room temperature. 1/100 volume of 400 mM magnesium acetate was added thereto to prepare a poly(rA)-poly(dT) solution.
8 $\mu$l of an appropriate dilution of an enzyme solution was added to 800 $\mu$l of the poly(rA)-poly(dT) solution. The mixture was reacted at 40°C for 20 minutes. The absorbance (A260) was measured over time to determine the change. Specifically, the concentration of nucleotide released from the poly (rA)-poly (dT) hybrid by the enzymatic reaction was determined on the basis of the difference in absorbance. One unit of an RNase H was defined as the amount of enzyme that increases A260 corresponding to the release of 1 nmol of ribonucleotide in 20 minutes calculated according to the following equation:

```
Unit = Change in absorbance per minute x 57.1 x
Dilution rate
```

Example 1

(1) Preparation of template RNA

An RNA transcript used as template for RT-ICAN was synthesized by in vitro transcription.
A plasmid DNA to be used as template for in vitro transcription was synthesized by an artificial synthetic gene preparation commission service (Takara Bio). A sequence from position 18133 to position 18362 (SEQ ID NO:1) of the genomic sequence of SARS (Severe Acute Respiratory Syndrome) coronavirus (GenBank Acc. No.: AY278741) was used. Recognition sequences for HindIII and BamHI for insertion into a plasmid were added at the 5' terminus and the 3' terminus of the sequence, respectively. This fragment was inserted between the HindIII site and the BamHI site in pBluescriptII SK(+). The resulting plasmid was cleaved with BamHI to obtain a linear DNA, which was used as template for in vitro transcription using T7 RNA polymerase.
MEGAscript T7 Kit (Ambion) was used for in vitro transcription. In vitro transcription, DNase I treatment and RNA purification were carried out according to the instructions attached to the kit, and the concentration of the synthesized RNA was measured on the basis of OD260. The RNA solution whose concentration was adjusted to $10^0$ to $10^5$ copies/$\mu$l with RNA Dilution Buffer (10 mM Tris-HCl (pH 7.5), 1 mM EDTA, 10 mM NaCl, 30 $\mu$g/ml *E. coli* 16S+23S rRNA (Boehringer Ingerheim)) was used as template for RT-ICAN.

(2) Examination of 2-step RT-ICAN

(A) Examination 1

The effect of attachment of a sequence that anneals to a region upstream of the second chimeric oligonucleotide primer to the 5' terminus of a reverse transcription primer was examined.
Changes in sensitivity and detection velocity of RT-ICAN by the use, as a reverse transcription primer, of a ladder-forming oligonucleotide primer having a 12-nucleotide sequence attached to the 5' terminus that anneals to a region upstream (-1 to -12) of the second chimeric oligonucleotide primer were studied.

The concentration of the template RNA prepared in Example 1-(1) was calculated on the basis of the OD260 value and adjusted to $10^0$, $10^1$, $10^2$, $10^3$, $10^4$ or $10^5$ copies per $\mu$l. A reaction mixture of 10 $\mu$l containing the following at final concentrations was prepared: 32 mM HEPES-KOH buffer (pH 7.8), 100 mM potassium acetate, 1% dimethyl sulfoxide, 0.11% BSA, 4 mM magnesium acetate, 500 $\mu$M of each dNTP, 1 pmol of primer 205RN3(18) (SEQ ID NO:2), primer A12-205R (SEQ ID NO:3), primer 215R (SEQ ID NO:4), primer A12-215R (SEQ ID NO:5) or primer A12-223R (SEQ ID NO:6) as reverse transcription primer, 12.5 U of RTase M-MLV (Takara Bio) and 1 $\mu$l of one of the template RNAs with varying copy numbers.

The reaction mixture was placed in Thermal Cycler Personal (Takara Bio), incubated at 45°C for 10 minutes, and then cooled to 4°C. After reverse transcription reaction, 15 $\mu$l of a reaction mixture containing the following at final concentrations was added to 10 $\mu$l of the reaction mixture: 32 mM HEPES-KOH buffer (pH 7.8), 100 mM potassium acetate, 1% dimethyl sulfoxide, 0.11% BSA, 4 mM magnesium acetate, 500 $\mu$M of each dNTP, 25 pmol each of primer 134FN3 (18) (SEQ ID NO:7) as the second chimeric oligonucleotide primer and primer 205RN3 (18) as the first chimeric oligonucleotide primer, 11 U of BcaBEST (Takara Bio), 0.05 U of Tli RNase HII prepared according to the method as described in Example 8 of WO 02/22831 and SYBR Green (Takara Bio). An ICAN reaction was carried out at 55°C using Rotor gene (Corbett Research) to detect the amplification product in a real-time manner. The reaction product was also detected by electrophoresis on a 3% agarose gel.

As a result, the sensitivity of RT-ICAN with reverse transcription using primer 205RN3 (18) was $10^4$ copies. When the reverse transcription primer A12-205R for the same position having a 12-nucleotide sequence attached to the 5' terminus that anneals to the region upstream (-1 to -12) of the second chimeric oligonucleotide primer was used, the sensitivity was $10^2$ copies, i.e., increased by two orders of magnitude. In addition, the detection velocity for $10^5$ copies was increased by about 2 minutes.

Similarly, the sensitivity of RT-ICAN with reverse transcription using the primer 215R DNA was $10^3$ copies. When the ladder-forming oligonucleotide primer A12-215R for the same position having a 12-nucleotide sequence attached to the 5' terminus that anneals to the region upstream (-1 to -12) of the second chimeric oligonucleotide primer was used for reverse transcription, the sensitivity was $10^0$ copy, i.e., increased by three orders of magnitude. In addition, the detection velocity for $10^5$ copies was increased by about 2 minutes.

Furthermore, when the ladder-forming oligonucleotide primer A12-223R having a 12-nucleotide sequence attached to the 5' terminus that anneals to the region upstream (-1 to -12) of the second chimeric oligonucleotide primer was used for reverse transcription, the sensitivity was $10^1$ copies.

As described above, increases in sensitivity and detection velocity were observed when a ladder-forming oligonucleotide primer having a 12-nucleotide sequence attached to the 5' terminus that anneals to a region upstream (-1 to -12) of the second chimeric oligonucleotide primer was used for reverse transcription, regardless of the portion of the template RNA to which the primer anneals.

(B) Examination 2

A similar examination was carried out using a ladder-forming oligonucleotide primer as reverse transcription primer while changing the position of the 12-nucleotide sequence (-1 to -12, -11 to -22, -21 to -32, +5 to -6, +11 to 0) attached to the 5' terminus that anneals to a region upstream of the second chimeric oligonucleotide primer and/or a part thereof.

First, the concentration of the template RNA prepared in Example 1-(1) was calculated on the basis of the OD260 value and adjusted to $10^0$, $10^1$, $10^2$, $10^3$, $10^4$ or $10^5$ copies per $\mu$l.

A reaction mixture of 10 $\mu$l containing the following at final concentrations was prepared: 32 mM HEPES-KOH buffer (pH 7.8), 100 mM potassium acetate, 1% dimethyl sulfoxide, 0.11% BSA, 4 mM magnesium acetate, 500 $\mu$M of each dNTP, 1 pmol of a ladder-forming oligonucleotide primer, primer 215R, primer A12-215R, primer A12(-10)-215R (SEQ ID NO:8), primer A12(-20)-215R (SEQ ID NO:9), primer A12(6)-215R (SEQ ID NO:10) or primer A12(12)-215R (SEQ ID NO:11), as reverse transcription primer, 50 U of RTase M-MLV (Takara Bio) and 1 $\mu$l of one of the template RNAs with varying copy numbers.

The reaction mixture was placed in Thermal Cycler Personal (Takara Bio), incubated at 45°C for 10 minutes, and then cooled to 4°C. After reverse transcription reaction, 15 $\mu$l of a reaction mixture containing the following at final concentrations was added to 10 $\mu$l of the reaction mixture: 32 mM HEPES-KOH buffer (pH 7.8), 100 mM potassium acetate, 1% dimethyl sulfoxide, 0.11% BSA, 4 mM magnesium acetate, 500 $\mu$M of each dNTP, 25 pmol each of primer B134FN3(16) (SEQ ID NO:12) as the second chimeric oligonucleotide primer and primer 205RN3(16) (SEQ ID NO:13) as the first chimeric oligonucleotide primer, 11 U of BcaBEST (Takara Bio), 0.05 U of Tli RNase HII and SYBR Green (Takara Bio). An ICAN reaction was carried out at 55°C using Rotor gene (Corbett Research) to detect the amplification product in a real-time manner. The reaction product was also detected by electrophoresis on a 3% agarose gel.

As a result, the sensitivity of RT-ICAN with reverse transcription using primer 215R was $10^2$ copies. When the ladder-forming oligonucleotide primer A12-215R for the same position having a 12-nucleotide sequence attached to the 5' terminus that anneals to the region upstream (-1 to -12) of the second chimeric oligonucleotide primer was used, the sensitivity was $10^1$ copies, i.e., increased by one order of magnitude. In addition, the detection velocity for $10^5$ copies was increased by about 3 minutes.

When the ladder-forming oligonucleotide primer A12(-10)-215R having a 12-nucleotide sequence attached to the 5' terminus that anneals to the region upstream (-11 to -22) of the second chimeric oligonucleotide primer was used, the sensitivity was $10^2$ copies, which was the same as that with primer 215R. The detection velocity for $10^5$ copies was increased by about 2 minutes. The detection of $10^2$ to $10^5$ copies was more quantitative (correlation coefficient: 0.997) than that with primer 215R.

When the ladder-forming oligonucleotide primer A12(-20)-215R having a 12-nucleotide sequence attached to the 5' terminus that anneals to the region upstream (-21 to -32) of the second chimeric oligonucleotide primer was used, the sensitivity was $10^1$ copies, i.e., increased by one order of magnitude. In addition, the detection velocity for $10^5$ copies was increased by about 2.5 minutes.

When the ladder-forming oligonucleotide primer A12(6)-215R having a 12-nucleotide sequence attached to the 5' terminus that anneals to the upstream region and a part of the second chimeric oligonucleotide primer, or the ladder-forming oligonucleotide primer A12(12)-215R having a 12-nucleotide sequence attached to the 5' terminus that anneals to a part of the second chimeric oligonucleotide primer was used, increases in detection sensitivity and detection velocity were also observed like in the case of the above-mentioned primer.

As described above, increases in sensitivity and detection velocity were observed using each ladder-forming oligonucleotide primer while changing the position of the 12-nucleotide sequence attached to the 5' terminus that anneals to a region upstream of the second chimeric oligonucleotide primer.

(C) Examination 3

Sensitivity and detection velocity of RT-ICAN were examined using a ladder-forming oligonucleotide primer having a 6-, 9- or 12-nucleotide sequence attached to the 5' terminus (-11 to -16, -11 to -19 or -11 to -22) that anneals to a region upstream of the second chimeric oligonucleotide primer as reverse transcription primer.

First, the concentration of the template RNA prepared in Example 1-(1) was calculated on the basis of the OD260 value and adjusted to $10^0$, $10^1$, $10^2$, $10^3$, $10^4$ or $10^5$ copies per $\mu$l.

A reaction mixture of 10 $\mu$l containing the following at final concentrations was prepared: 32 mM HEPES-KOH buffer (pH 7.8), 100 mM potassium acetate, 1% dimethyl sulfoxide, 0.11% BSA, 4 mM magnesium acetate, 500 $\mu$M of each dNTP, 1 pmol of a ladder-forming oligonucleotide primer, primer 215R, primer A6(-10)-215R (SEQ ID NO:14), primer A9(-10)-215R (SEQ ID NO:15) or primer A12(-10)-215R, as reverse transcription primer, 50 U of RTase M-MLV (Takara Bio) and 1 $\mu$l of one of the template RNAs with varying copy numbers.

The reaction mixture was placed in Thermal Cycler Personal (Takara Bio), incubated at 45°C for 10 minutes, and then cooled to 4°C. After reverse transcription reaction, 15 $\mu$l of a reaction mixture containing the following at final concentrations was added to 10 $\mu$l of the reaction mixture: 32 mM HEPES-KOH buffer (pH 7.8), 100 mM potassium acetate, 1% dimethyl sulfoxide, 0.11% BSA, 4 mM magnesium acetate, 500 $\mu$M of each dNTP, 25 pmol each of primer B134FN3(16) as the second chimeric oligonucleotide primer and primer 205RN3(16) as the first chimeric oligonucleotide primer, 11 U of BcaBEST (Takara Bio), 0.05 U of Tli RNase HII and SYBR Green. An ICAN reaction was carried out at 55°C using Rotor gene (Corbett Research) to detect the amplification product in a real-time manner. The reaction product was also detected by electrophoresis on a 3% agarose gel.

As a result, the sensitivity of RT-ICAN with reverse transcription using primer 215R was $10^3$ copies. When the ladder-forming oligonucleotide primer A6(-10)-215R for the same position having a 6-nucleotide sequence attached to the 5' terminus that anneals to the region upstream (-11 to -16) of the second chimeric oligonucleotide primer was used, the sensitivity was $10^2$ copies, i.e., increased by one order of magnitude. In addition, the detection velocity for $10^5$ copies was increased by about 1 minute.

When the ladder-forming oligonucleotide primer A9(-10)-215R having a 9-nucleotide sequence attached to the 5' terminus that anneals to the region upstream (-11 to -19) of the second chimeric oligonucleotide primer was used, the sensitivity was $10^1$ copies, i.e., increased by two orders of magnitude. In addition, the detection velocity for $10^5$ copies was increased by about 2.5 minutes.

When the ladder-forming oligonucleotide primer A12(-10)-215R having a 12-nucleotide sequence attached to the 5' terminus that anneals to the region upstream (-11 to -22) of the second chimeric oligonucleotide primer was used, the sensitivity was $10^1$ copies, i.e., increased by two orders of magnitude. In addition, the detection velocity for $10^5$ copies was increased by about 3 minutes.

As described above, among the ladder-forming oligonucleotide primers with varying numbers of attached nucleotides, increases in sensitivity and detection velocity were observed using a ladder-forming oligonucleotide primer having a sequence of 6 nucleotides or more attached to the 5' terminus that anneals to a region upstream of the second chimeric oligonucleotide primer. It was confirmed that a longer sequence of nucleotides that anneals to a region upstream of the second chimeric oligonucleotide primer results in a greater effect.

(D) Examination 4

Detection velocity and quantitativeness of RT-ICAN were examined using A12-215R, a ladder-forming oligonucleotide primer having a 12-nucleotide sequence attached to the 5' terminus that anneals to a region upstream (-1 to -12) of the second chimeric oligonucleotide primer.
First, the concentration of the template RNA prepared in Example 1-(1) was calculated on the basis of the OD260 value and adjusted to $10^0$, $10^1$, $10^2$, $10^3$, $10^4$ or $10^5$ copies per $\mu$l.
A reaction mixture of 10 $\mu$l containing the following at final concentrations was prepared: 32 mM HEPES-KOH buffer (pH 7.8), 100 mM potassium acetate, 1% dimethyl sulfoxide, 0.11% BSA, 4 mM magnesium acetate, 500 $\mu$M of each dNTP, 1 pmol of the primer A12-215R, 50 U of RTase M-MLV (Takara Bio) and 1 $\mu$l of one of the template RNAs with varying copy numbers.
The reaction mixture was placed in Thermal Cycler Personal (Takara Bio), incubated at 45°C for 10 minutes, and then cooled to 4°C. After reverse transcription reaction, 15 $\mu$l of a reaction mixture containing the following at final concentrations was added to 10 $\mu$l of the reaction mixture: 32 mM HEPES-KOH buffer (pH 7.8), 100 mM potassium acetate, 1% dimethyl sulfoxide, 0.11% BSA, 4 mM magnesium acetate, 500 $\mu$M of each dNTP, 25 pmol each of primer B139FN3(16) as the second chimeric oligonucleotide primer and primer 205RN3(16) as the first chimeric oligonucleotide primer, 11 U of BcaBEST (Takara Bio), 0.05 U of Tli RNase HII and SYBR Green. An ICAN reaction was carried out at 55°C using Rotor gene (Corbett Research) to detect the amplification product in a real-time manner at every cycle (1 minute).
Amplification curves are shown in Figure 2. As shown in Figure 2, $10^1$ to $10^5$ copies were quantitatively detected (correlation coefficient: 0.992). Progress of the reaction for down to 10 copies was observed within 10 cycles.
Furthermore, amplification products were also observed according to a probe hybridization method by development on chromatography strips.
Observation of amplification products according to a probe hybridization method by development on chromatography strips was carried out using the Detection Set contained in the TaKaRa Bed-Side ICAN bla$^{IMP}$ Detection Kit (Takara Bio) according to the detection method described in the attached instructions. In the detection, a probe solution containing the FITC-labeled probe SARS-BNI-B (SEQ ID NO:16) was used as a probe, while the blaMP Probe Solution and the IC Probe Solution contained in the kit were not used.
As a result, development of reddish purple lines was observed for the amplification products from the reactions with $10^0$ to $10^5$ copies, indicating specific amplification.

(3) Examination of 1-step RT-ICAN

(A) Examination 1

The effect of the use of a first chimeric oligonucleotide primer having a sequence attached to the 5' terminus that anneals to a region upstream of the second chimeric oligonucleotide primer as ladder-forming oligonucleotide primer was examined.
Sensitivity and detection velocity of RT-ICAN were examined using a first chimeric oligonucleotide primer having a 7-nucleotide sequence attached to the 5' terminus that anneals to a region upstream (-1 to -7) of the second chimeric oligonucleotide primer. As a result of the attachment of the 7-nucleotide sequence to the 5' terminus of the first chimeric oligonucleotide primer, a sequence of 12 nucleotides from the 5' terminus of the first chimeric oligonucleotide primer constituted a sequence that anneals to a region upstream (-1 to -12) of the second chimeric oligonucleotide primer. A reverse transcription reaction and an ICAN reaction were carried out in parallel in the same tube.
First, the concentration of the template RNA prepared in Example 1-(1) was calculated on the basis of the OD260 value and adjusted to $10^0$, $10^1$, $10^2$, $10^3$, $10^4$ or $10^5$ copies per $\mu$l. 1 $\mu$l of one of the template RNAs with varying copy numbers was added to a reaction mixture of 24 $\mu$l containing the following at final concentrations: 32 mM HEPES-KOH buffer (pH 7.8), 100 mM potassium acetate, 1% dimethyl sulfoxide, 0.11% BSA, 4 mM magnesium acetate, 500 $\mu$M of each dNTP, 25 pmol each of primer 160FN3 (SEQ ID NO:17) as the second chimeric oligonucleotide primer and primer 241RNA3 (SEQ ID NO:18) as the first chimeric oligonucleotide primer, or primer (A12) 241RN3 (SEQ ID NO:19) as the first chimeric oligonucleotide primer, 50 U of RTase M-MLV (Takara Bio), 11 U of BcaBEST (Takara Bio), 0.05 U of Tli RNase HII and SYBR Green (Takara Bio). The reaction mixture was incubated at 45°C for 5 minutes and at 55°C for 45 minutes using Rotor gene (Corbett Research) to detect the amplification product in a real-time manner. The reaction product was also detected by electrophoresis on a 3% agarose gel.
As a result, the sensitivity of RT-ICAN using primer 241RN3 as the first chimeric oligonucleotide primer was $10^5$ copies. When primer (A12)241RN3 having a 7-nucleotide sequence attached to the 5' terminus that anneals to the region upstream (-1 to -7) of the second chimeric oligonucleotide primer was used, the sensitivity was $10^3$ copies, i.e., increased by two orders of magnitude. In addition, the detection velocity for $10^5$ copies was increased by about 1 minute.

As described above, increases in sensitivity and detection velocity were observed using a first chimeric oligonucleotide primer having a sequence attached to the 5' terminus that anneals to a region upstream of the second chimeric oligonucleotide primer as ladder-forming oligonucleotide primer.

(B) Examination 2

The effect, on a 1-step RT-ICAN reaction, of a ladder-forming oligonucleotide primer having a sequence attached to the 5' terminus that anneals to a region upstream of the second chimeric oligonucleotide primer was examined.

A ladder-forming oligonucleotide primer having a 12-nucleotide sequence attached to the 5' terminus that anneals to a region upstream (-1 to -12) of the second chimeric oligonucleotide primer was added to a 1-step RT-ICAN reaction system in which a reverse transcription reaction and an ICAN reaction were carried out in parallel in the same tube, and the sensitivity and the detection velocity were examined.

First, the concentration of the template RNA prepared in Example 1-(1) was calculated on the basis of the OD260 value and adjusted to $10^0$, $10^1$, $10^2$, $10^3$, $10^4$ or $10^5$ copies per μl. 1 μl of one of the template RNAs with varying copy numbers was added to a reaction mixture of 24 μl containing the following at final concentrations: 32 mM HEPES-KOH buffer (pH 7.8), 100 mM potassium acetate, 1% dimethyl sulfoxide, 0.11% BSA, 4 mM magnesium acetate, $500\mu$ M of each dNTP, 25 pmol each of primer 134FN3 (16) (SEQ ID NO:20) as the second chimeric oligonucleotide primer and primer 205RN3(16) as the first chimeric oligonucleotide primer, 50 U of RTase M-MLV (Takara Bio), 11 U of BcaBEST (Takara Bio), 0.05 U of Tli RNase HIT and SYBR Green (Takara Bio), or a reaction mixture of 24 μl further containing 2.5 pmol of primer A12-223R as ladder-forming oligonucleotide primer. The reaction mixture was incubated at 45°C for 5 minutes and at 55°C for 45 minutes using Rotor gene (Corbett Research) to detect the amplification product in a real-time manner. The reaction product was also detected by electrophoresis on a 3% agarose gel.

As a result, the sensitivity of the 1-step RT-ICAN without the addition of primer A12-223R as ladder-forming oligonucleotide primer was $10^3$ copies. When primer A12-223R was added, the sensitivity was $10^2$ copies, i.e., increased by one order of magnitude. In addition, the detection velocity for $10^3$ copies was increased by about 4 minutes.

As described above, increases in sensitivity and detection velocity were observed as a result of the addition of a ladder-forming oligonucleotide primer to a 1-step RT-ICAN reaction.

Example 2

(1) Examination of the effect on the detection of a polymorphism in human aldehyde dehydrogenase 2 gene (ALDH2) using a real-time detection system according to the cycling probe method

The effect on the detection of a polymorphism by real-time detection using the ICAN reaction and the cycling probe method was examined. The gene encoding the enzyme human aldehyde dehydrogenase 2 (GenBank Acc. No.: AH002599) was selected as the subject to be detected. It has been reported that a single nucleotide polymorphism in which glutamic acid at position 487 (GAA) is replaced by lysine (AAA) exists in exon 12 of this gene, and is profoundly related to the individual difference in predisposition concerning alcohol-drinking and involved in the risk of carcinogenesis.

Primer ICAN-ALDH2-F (SEQ ID NO:21) which corresponds to the second chimeric oligonucleotide primer and primer ICAN-ALDH2-R (SEQ ID N0:22) which corresponds to the first chimeric oligonucleotide primer were synthesized using a DNA synthesizer (Applied Biosystems) in order to detect the polymorphism in the aldehyde dehydrogenase 2 gene in an ICAN reaction system. A probe for detecting the normal type ALDH2 wG (SEQ ID NO:23) and a probe for detecting the mutant type ALDH2 mA (SEQ ID NO:24) were synthesized. The probe for detecting the normal type ALDH2 wG is a DNA-RNA-DNA-type oligonucleotide probe having a ROX label (Applied Biosystems) as fluorescent label attached to the 5' terminus and Eclipse (Epoch Biosciences) as quenching label attached to the 3' terminus. The probe for detecting the mutant type ALDH2 mA is a DNA-RNA-DNA-type oligonucleotide probe having a FAM label (Applied Biosystems) as fluorescent label attached to the 5' terminus and Eclipse as quenching label attached to the 3' terminus.

In addition, the following ladder-forming oligonucleotide primers each having a 12-nucleotide sequence attached to the 5' terminus that anneals to a region upstream (-1 to -12) of the second chimeric oligonucleotide primer which were designed around the primer ICAN-ALDH2-R were synthesized: ALDH2-TH1 (SEQ ID NO:25; -1 to +15 relative to the chimeric oligonucleotide primer ICAN-ALDH2-R); ALDH2-TH2 (SEQ ID NO:26; -9 to +6 relative to the chimeric oligonucleotide primer ICAN-ALDH2-R); and ALDH2-TH3 (SEQ ID NO:27; -17 to -2 relative to the chimeric oligonucleotide primer ICAN-ALDH2-R).

Genomic DNAs as templates were prepared using the QIAamp DNA Mini Kit (Qiagen) from blood samples collected after obtaining informed consent for which EDTA was used as anticoagulant.

The reaction conditions for the ICAN reaction were as follows. Briefly, a reaction mixture of a final volume of 25 μl containing the following at final concentrations was prepared: 32 mM HEPES-KOH buffer (pH 7.8), 100 mM potassium acetate, 5 mM magnesium acetate, 1% dimethyl sulfoxide, 0.04% propylenediamine, 0.11% bovine serum albumin, 600 $\mu$M of each dNTP, 4 U of BcaBEST DNA polymerase, 100 U of Tli RNase HII, 50 pmol each of primer ICAN-ALDH2-F and primer ICAN-ALDH2-R, 5.5 pmol of the probe ALDH2 wG, 6 pmol of the probe ALDH2 mA, 100 ng of genomic DNA as template and sterile water with or without the addition of 1 pmol of the ladder-forming oligonucleotide primer ALDH2-TH1, ALDH2-TH2 or ALDH2-TH3. The reaction mixture was placed in Smart Cycler (Takara Bio), treated at 70°C for 5 minutes and incubated at 56°C for 60 minutes. The fluorescence intensity was measured during the incubation at 56°C at 1-minute intervals.

Furthermore, a PCR was conducted using genomic DNA as template and a pair of primers, ALDH2-F (SEQ ID NO:28) and ALDH2-R (SEQ ID NO:29). The resulting amplification product was purified using Microcon-30 (Millipore), digested with Eco57I and subjected to electrophoresis on a 3% NuSieve 3:1 agarose gel (Takara Bio) for RFLP typing.

The PCR was conducted using TaKaRa ExTaq (Takara Bio). Specifically, a reaction mixture of a final volume of 50 $\mu$l containing the following at final concentrations was prepared: 1 x ExTaq buffer, 200 $\mu$M of each dNTP, 1.25 U of ExTaq, 10 pmol each of primer ALDH2-F and primer ALDH2-R, 5 $\mu$l of human genomic DNA prepared as a template nucleic acid and sterile water. The reaction mixture was placed in Thermal Cycler SP (Takara Bio) and subjected to heating at 94°C for 30 seconds followed by 30 cycles of 94°C for 30 seconds, 55°C for 30 seconds and 72°C for 30 seconds.

Eco57I is a restriction enzyme that recognizes CTGAAG. It cleaves the amplification product from the normal type aldehyde dehydrogenase 2, but does not cleave the amplification product from the mutant type.

When a genomic DNA sample for which three fragments (cleaved and uncleaved amplification product) were observed upon PCR RFLP with ECo57I (i.e., which was considered to be heterozygotic) was used, decrease in Ct value was observed upon addition of ALDH2-TH1, ALDH2-TH2 or ALDH2-TH3 to the reaction system as shown in Tables 1 and 2 below, indicating increase in reactivity. The Ct value is the point at which an amplification curve for a sample intersects a threshold line (100). When ALDH2-THI or ALDH2-TH2 was added (not in case of ALDH2-TH3), an increase in fluorescence intensity from FAM was observed, indicating an increase in detection efficiency.

[Table 1]

Table 1: Change in Ct value

|  | No addition | ALDH2-TH1 | ALDH2-TH2 | ALDH2-TH3 |
|---|---|---|---|---|
| ROX (wild) | 30.3-33.03 | 21.01-23.87 | 21.06-27.37 | 22.91-24.05 |
| FAM (mutant) | 36.45-40.44 | 22.9-28.0 | 25.65-27.98 | 24.83-34.71 |

[Table 2]

Table 2: Fluorescence intensity

|  | No addition | ALDH2-TH1 | ALDH2-TH2 | ALDH2-TH3 |
|---|---|---|---|---|
| ROX (wild) | 350-340 | 330-350 | 350-370 | 340-380 |
| FAM (mutant) | 150-245 | 285-370 | 370-380 | 145-250 |

When a genomic DNA sample for which two fragments (cleaved amplification product) were observed upon RFLP with Eco57I (i.e., which was considered to be homozygotic for the normal type) was used, only ROX fluorescence signals from the probe ALDH2 wG were observed. When a genomic DNA sample for which one fragment (uncleaved amplification product) was observed upon RFLP with Eco57I (i.e., which was considered to be homozygotic for the mutant type) was used, only 6-FAM fluorescence signals from the probe ALDH2 mA were observed. In these cases, decreases in Ct values were observed, indicating an increase in reactivities. The Ct value is the point at which an amplification curve for a sample intersects a threshold line (100).

(2) Examination of the effect of a nucleotide sequence attached to the 5' terminus of the ladder-forming oligonucleotide primer added to the reaction system on the detection of a polymorphism in ALDH2

Regarding the effect on the detection of a polymorphism by real-time detection using the ICAN reaction and the cycling probe method as described in Example 2-(1), the effect of a nucleotide sequence attached to the 5' terminus of the ladder-forming oligonucleotide primer to be added to the reaction system on the detection of the gene encoding the enzyme.human aldehyde dehydrogenase 2, which was selected as the subject to be detected, was examined.

Like in Example 2-(1), the primer ICAN-ALDH2-F, the primer ICAN-ALDH2-R, the probe for detecting the normal type ALDH2 wG and the probe for detecting the mutant type ALDH2 mA were used for detecting a polymorphism in the aldehyde dehydrogenase 2 gene using an ICAN reaction system. The probe for detecting the normal type ALDH2 wG is a DNA-RNA-DNA-type oligonucleotide probe having a ROX label as fluorescent label attached to the 5' terminus and Eclipse as quenching label attached to the 3' terminus. The probe for detecting the mutant type ALDH2 mA is a DNA-RNA-DNA-type oligonucleotide probe having a FAM label as fluorescent label attached to the 5' terminus and Eclipse as quenching label attached to the 3' terminus.

In addition, the ladder-forming oligonucleotide primer ALDH2-TH2 (-9 to +6 relative to the chimeric primer ICAN-ALDH2-R) having a 12-nucleotide sequence attached to the 5' terminus that anneals to a region upstream (-1 to -12) of the primer ICAN-ALDH2-F which was designed around the primer ICAN-ALDH2-R, and primer ALDH2-TH4 (SEQ ID NO:30) in which the 12-nucleotide sequence attached to the 5' terminus of the primer ALDH2-TH2 is removed were synthesized.

Genomic DNAs as templates were prepared using the QIAamp DNA Mini Kit (Qiagen) from blood samples collected after obtaining informed consent for which EDTA was used as anticoagulant, and one genomic DNA which was confirmed to be heterozygotic by RFLP typing was used.

The reaction conditions for the ICAN reaction were as follows. Briefly, a reaction mixture of a final volume of 25 μl containing the following at final concentrations was prepared: 32 mM HEPES-KOH buffer (pH 7.8), 100 mM potassium acetate, 5 mM magnesium acetate, 1% dimethyl sulfoxide, 0.04% propylenediamine, 0.11% bovine serum albumin, 600 μM of each dNTP, 4 U of BcaBEST DNA polymerase, 100 U of Tli RNase HII, 50 pmol each of the primers ICAN-ALDH2-F and ICAN-ALDH2-R, 5.5 pmol of the probe ALDH2 wG, 6 pmol of the probe ALDH2 mA, 100 ng of the genomic DNA as a template and sterile water with or without the addition of 1 pmol of the ladder-forming oligonucleotide primer ALDH2-TH2 or ALDH2-TH4. The reaction mixture was placed in Smart Cycler (Takara Bio), treated at 70°C for 5 minutes and incubated at 56°C for 60 minutes. The fluorescence intensity was measured during the incubation at 56°C at 1-minute intervals.

A decrease in Ct value was observed upon addition of ALDH2-TH2 to the reaction system as shown in Tables 3 and 4 below, indicating an increase in reactivity. The Ct value is the point at which an amplification curve for a sample intersects a threshold line (100). Furthermore, increases in fluorescence intensities from ROX and FAM were observed, indicating an increase in detection efficiency. On the other hand, a change in Ct value or fluorescence intensity was not observed when ALDH2-TH4 was added to the reaction system. That is, when ALDH2-TH4 lacking the 12-nucleotide sequence that anneals to the region upstream (-1 to -12) of the primer ICAN-ALDH2-F, which was attached to the 5' terminus of ALDH-TH2, was added to the reaction system, the increases in reactivity and detection efficiency which were observed for ALDH2-TH2 were not observed.

[Table 3]

Table 3: Chancre in Ct value

|  | No addition | ALDH2-TH2 | ALDH2-TH4 |
|---|---|---|---|
| ROX (wild) | 28.2 | 23.9 | 27.2 |
| FAM (mutant) | 34.08 | 24.7 | 34.8 |

[Table 4]

Table 4: Fluorescence intensity

|  | No addition | ALDH2-TH2 | ALDH2-TH4 |
|---|---|---|---|
| ROX (wild) | 310-320 | 410-420 | 350-360 |
| FAM (mutant) | 270-290 | 465-475 | 230-240 |

(3) Examination of the effect of a nucleotide sequence attached to the 5' terminus of the ladder-forming oligonucleotide primer added to the reaction system on the detection of *Legionella*

A ladder-forming oligonucleotide primer having a 12-nucleotide sequence attached to the 5' terminus that anneals to a region upstream (-1 to -12 or -13 to -24) of the second chimeric oligonucleotide primer was added, and the change in detection velocity of the ICAN reaction was examined.

The effect on real-time detection using the ICAN reaction and the cycling probe method was examined. A gene encoding *Legionella* Mip (*Legionella pneumophila*, macrophage infectivity potentiator (Mip); GenBank Acc. No.: AF095215) was selected as the subject to be detected.

The chimeric oligonucleotide primer F2 (SEQ ID NO:31) as the second chimeric oligonucleotide primer and the chimeric oligonucleotide primer R2 (SEQ ID NO:32) as the first chimeric oligonucleotide primer were synthesized using a DNA synthesizer (Applied Biosystems) in order to detect the *Legionella* Mip gene using an ICAN reaction system. A probe for detecting the Mip gene, Mip4g12 (SEQ ID NO:33), was synthesized. The probe for detecting the Mip gene, Mip4g12, is a DNA-RNA-DNA-type oligonucleotide probe having a FAM label as fluorescent label attached to the 5' terminus and Eclipse as quenching label attached to the 3' terminus.

In addition, the following ladder-forming oligonucleotide primers each having a 12-nucleotide sequence attached to the 5' terminus that anneals to a region upstream (-1 to -12 or -13 to -24) of the second chimeric oligonucleotide primer which were designed around the first chimeric oligonucleotide primer were synthesized: R2(-13) (SEQ ID NO:34; -13 to -29 relative to the first chimeric oligonucleotide primer); R2(-13)A12-1 (SEQ ID NO:35; -13 to -29 relative to the first chimeric oligonucleotide primer with a 12-nucleotide sequence of a region upstream (-1 to -12) of the second chimeric oligonucleotide primer attached to the 5' terminus); and R2(-13)A12-2 (SEQ ID NO:36; -13 to -29 relative to the first chimeric oligonucleotide primer with a 12-nucleotide sequence of a region upstream (-13 to -24) of the second chimeric oligonucleotide primer attached to the 5' terminus).

*Legionella pneumophila* Control DNA contained in the EnviroAmp™ kit (Perkin Elmer) was used as genomic DNA as a template.

The reaction conditions for the ICAN reaction were as follows. Briefly, a reaction mixture of a final volume of 25 µl containing the following at final concentrations was prepared: 32 mM HEPES-KOH buffer (pH 7.8), 100 mM potassium acetate, 5 mM magnesium acetate, 1% dimethyl sulfoxide, 0.11% bovine serum albumin, 500 µM of each dNTP, 2 U of BcaBEST DNA polymerase, 100 U of Tli RNase HII, 25 pmol each of the chimeric oligonucleotide primer F2 and the chimeric oligonucleotide primer R2, 5 pmol of the probe Mip4g12, 200 copies of Control DNA as a template and sterile water with or without the addition of 1 pmol of R2(-13)A12-1 or R2(-13)A12-2. The reaction mixture was placed in Smart Cycler (Takara Bio), treated at 70°C for 5 minutes and incubated at 53°C for 90 minutes. The fluorescence intensity was measured during the incubation at 53°C at 1-minute intervals.

The results are shown in Table 5. A decrease in Ct value was observed upon addition of the ladder-forming oligonucleotide primer R2(-13)A12-1 or R2(-13)A12-2 to the reaction system as shown in Table 5, indicating an increase in reactivity. The Ct value is the point at which an amplification curve for a sample intersects a threshold line (100).

[Table 5]

Table 5: Change in Ct value

|  | No addition | R12(-13)A12-1 | R12(-13)A12-2 |
|---|---|---|---|
| FAM | 74.17-81.12 | 44.11-54.61 | 48.03-55.89 |

Example 3

(1) Effect of complementary sequences in regions in the nucleic acid as template upstream of the respective ICAN primers on the ICAN reaction (1)

6-nucleotide complementary sequences were inserted into regions in a nucleic acid as a template upstream of the 5' termini of the respective ICAN primers, and the effect on the ICAN reaction was examined. Human c-Ki-ras 2 gene (GenBank Acc. No.: L00045) was selected as the subject to be detected.

A template without definite complementary sequences in regions upstream of the 5' termini of the respective ICAN primers, and a template with 6-nucleotide complementary sequences were prepared as follows. PCRs were conducted using Human genome (purchased from Clontech) as a template as well as primer c-Ki-ras/12F (SEQ ID NO:37) and primer rasT1R (SEQ ID NO:38), or primer-rasT14F (SEQ ID NO:39) and primer rasT4R (SEQ ID NO:40) which are primers having complementary sequences attached at the 5' termini of the respective primers. The resulting fragments were blunted, and inserted into a HincII site an plasmid pUC118 (Takara Bio). Plasmids in which the direction of the primer c-Ki-ras/12F or the primer rasT14F was the same as that of the M13 primer M4 (Takara Bio) which anneals to pUC118 were selected from the resulting plasmids. As a result, plasmid T7 without definite complementary sequences in regions upstream of the 5' termini of the respective ICAN primers and plasmid T16 with 6-nucleotide complementary sequences CGCGCG were obtained.

The concentration of the prepared plasmid DNA was calculated on the basis of the OD260 value and adjusted to $10^0$, $10^1$, $10^2$ or $10^3$ copies per µl. 1 µl of one of the template DNAs with varying copy numbers was added to a reaction mixture of 24 µl containing the following at final concentrations: 32 mM HEPES-KOH buffer (pH 7.8), 100 mM potassium acetate, 1% dimethyl sulfoxide, 0.11% BSA, 4 mM magnesium acetate, 500 µM of each dNTP, 30 pmol each of the chimeric oligonucleotide primer c-Ki-ras/12FN3 (SEQ ID NO:41) and the chimeric oligonucleotide primer c-Ki-ras/12RN3 (SEQ ID NO:42), 2.8 U of BcaBEST (Takara Bio) and 2.2 U of Afu RNase HII prepared according to the method as described in Example 7 of WO 02/22831. The reaction mixture was incubated at 55°C for 60 minutes using Thermal Cycler Personal (Takara Bio). The amplification product was detected by electrophoresis on a 3% agarose gel.

As a result, when the plasmid T7 without definite complementary sequences in regions upstream of the 5' termini of the respective ICAN primers was used as a template, only a band corresponding to the size of the target region was observed as amplification product with stable sensitivity of $10^3$ copies. On the other hand, when the plasmid T16 with the sequence CGCGCG which is complementary to upstream regions adjacent to the 5' termini of the respective ICAN primers was used as a template, a band corresponding to the size of the target region as well as high-molecular-weight products in a regular ladder-like pattern were observed with stable sensitivity of 10 copies, indicating an increase in sensitivity. The ladder-like product was excised from the gel, subcloned into a HincII site in pUC118, and subjected to nucleotide sequence determination. Then, it was found that in the ladder-like product the target regions were connected to each other in the same direction through the complementary sequence CGCGCG in regions upstream of the 5' termini of the respective ICAN primers.

As described above, a reaction of polymerizing target regions was promoted due to complementary sequences in regions upstream of the '5' termini of the respective ICAN primers, resulting in an increases in sensitivity.

(2) Effect of complementary sequences in regions in the nucleic acid as template upstream of the respective ICAN primers on the ICAN reaction (2)

The effect of 3-nucleotide complementary sequences in regions in the nucleic acid as a template upstream of the 5' termini of the respective ICAN primers on the ICAN reaction was examined. Gonococcus cppB (Neisseria gonorrhoeae cryptic plasmid protein (cppB); GenBank Acc. No.: M10316) gene was selected as the subject to be detected.

A template was prepared as follows.

A PCR was conducted using the gonococcus genome (purchased from Summit Pharmaceuticals International Corporation) as a template as well as primer PJDBF (SEQ ID NO:43) and primer PJDBR (SEQ ID NO:44). The resulting fragment was blunted, and inserted into a HincII site in plasmid pUC118 (Takara Bio). A plasmid in which the direction of the primer PJDBF was the same as that of the M13 primer M4 (Takara Bio) which anneals to pUC118 was selected from the resulting plasmids. As a result, plasmid Cp13 was obtained.

Primer PJDBOFN3 (SEQ ID NO:45) and primer PJDBORN3 (SEQ ID NO:46) were designed in order to amplify the inserted region using plasmid Cp13 as a template. In this case, the strand of the target region for primer PJDBOFN3 did not contain the sequence GTC which is a sequence complementary to the upstream 3-nucleotide sequences GAC adjacent to the 5' terminus of primer PJDBORN3. The sequence existed at a position 34 nucleotides upstream of the 5' terminus of primer PJDBOFN3. The effect of the 3-nucleotide sequence on ICAN was examined.

The concentration of the plasmid DNA prepared as described above was calculated on the basis of the OD260 value and adjusted to $10^0$, $10^1$, $10^2$, $10^3$, $10^4$, $10^5$, $10^6$, $10^7$ or $10^8$ copies per $\mu$l. 1 $\mu$l of one of the template DNAs with varying copy numbers was added to a reaction mixture of 24 $\mu$l containing the following at final concentrations: 32 mM HEPES-KOH buffer (pH 7.8), 100 mM potassium acetate, 1% dimethyl sulfoxide, 0.11% BSA, 4 mM magnesium acetate, 500 $\mu$M of each dNTP, 30 pmol each of the chimeric oligonucleotide primer PJDBOFN3 and the chimeric oligonucleotide primer PJDBORN3, 2.8 U of BcaBEST (Takara Bio) and 2.2 U of Afu RNase HII. The reaction mixture was incubated at 55°C for 60 minutes using Thermal Cycler Personal (Takara Bio). The amplification product was detected by electrophoresis on a 3% agarose gel.

As a result, a band corresponding to the size of the target region as well as high-molecular-weight products in a regular ladder-like pattern were observed with high sensitivity ($10^2$ copies). The ladder-like product was excised from the gel, subcloned into a HincII site in pUC118, and subjected to nucleotide sequence determination. Then, it was found that in the ladder-like product the target regions were connected to each other in the same direction in a regular pattern through the 3-nucleotide complementary sequences GAC and GTC in regions upstream of the 5' termini of the respective ICAN primers. The ladder-like amplification product contained the target region between the ICAN primers and the 34-nucleotide region upstream of the 5' terminus of the chimeric oligonucleotide primer PJDBOFN3 between the target regions in a regular pattern.

As described above, it was confirmed that a reaction of polymerizing target regions was promoted due to 3-nucleotide complementary sequences in regions upstream of the 5' termini of the respective ICAN primers, enabling highly sensitive amplification. In addition, it was shown that if an amplification product is to be detected using hybridization with a probe, probe positions can be selected from a region between respective ICAN primers or a region between an ICAN primer and a complementary sequence in a region upstream of the 5' terminus of an ICAN primer.

Example 4

Sensitivity and detection velocity of RT-ICAN were examined using a ladder-forming oligonucleotide primer having a 6-, 9- or 12-nucleotide sequence attached to the 5' terminus that anneals to a region upstream (-1 to -6, -1 to -9 or -1 to -12) of the second chimeric oligonucleotide primer as reverse transcription primer.

First, the concentration of the template RNA prepared in Example 1-(1) was calculated on the basis of the OD260 value and adjusted to $10^0$, $10^1$, $10^2$, $10^3$, $10^4$ or $10^5$ copies per $\mu$l.

A reaction mixture of 10 $\mu$l containing the following at final concentrations was prepared: 32 mM HEPES-KOH buffer (pH 7.8), 100 mM potassium acetate, 1% dimethyl sulfoxide, 0.11% BSA, 4 mM magnesium acetate, 500 $\mu$M of each dNTP, 1 pmol of the ladder-forming oligonucleotide primer 215R, primer A6-215R (SEQ ID NO:47), primer A9-215R (SEQ ID NO:48) or primer A12-215R as reverse transcription primer, 50 U of RTase M-MLV (Takara Bio) and 1 $\mu$l of one of the template RNAs with varying copy numbers.

The reaction mixture was placed in Thermal Cycler Personal (Takara Bio), incubated at 45°C for 10 minutes, and then cooled to 4°C. After reverse transcription reaction, 15 $\mu$l of a reaction mixture containing the following was added to 10 $\mu$ of the reaction mixture: mM potassium acetate, 1% dimethyl sulfoxide, 0.11% BSA, 4 mM magnesium acetate, 500 $\mu$M of each dNTP, 25 pmol each of primer B134FN3(16) as the second chimeric oligonucleotide primer and primer 205RN3(16) as the first chimeric oligonucleotide primer, 11 U of BcaBEST (Takara Bio), 0.05 U of Tli RNase HII and SYBR Green. An ICAN reaction was carried out at 55°C using Rotor gene (Corbett Research) to detect the amplification product in a real-time manner. The reaction product was also detected by electrophoresis on a 3% agarose gel.

When the ladder-forming oligonucleotide primer A6-215R having a 6-nucleotide sequence attached to the 5' terminus that anneals to a region upstream (-1 to -6) of the second chimeric oligonucleotide primer was used, the sensitivity was $10^2$ copies. The attachment of the 6-nucleotide sequence increased the detection velocity by 0.9 minutes. When the ladder-forming oligonucleotide primer A9-215R having a 9-nucleotide sequence attached to the 5' terminus that anneals to a region upstream (-1 to -9) of the second chimeric oligonucleotide primer was used, the sensitivity was $10^2$ copies. The attachment of the 9-nucleotide sequence increased the detection velocity by 1.6 minutes.
When the ladder-forming oligonucleotide primer A12-215R having a 12-nucleotide sequence attached to the 5' terminus that anneals to a region upstream (-1 to -12) of the second chimeric oligonucleotide primer was used, the sensitivity was $10^1$ copies. The attachment of the 12-nucleotide sequence increased the detection velocity by 3.7 minutes.

As described above, it was confirmed that a longer sequence of nucleotides attached to the 5' terminus of a ladder-forming oligonucleotide primer that anneals to a region upstream of the second chimeric oligonucleotide primer results in a greater effect.

Example 5

Sensitivity of RT-ICAN was examined using a ladder-forming oligonucleotide primer having a 12- or 18-nucleotide sequence attached to the 5' terminus that anneals to a region upstream (-1 to -12 or -1 to -18) of the second chimeric oligonucleotide primer as reverse transcription primer.

First, the concentration of the template RNA prepared in Example 1-(1) was calculated on the basis of the 0D260 value and adjusted to $10^0$, $10^1$, $10^2$, $10^3$, $10^4$ or $10^5$ copies per $\mu$l.
A reaction mixture of 10 $\mu$l containing the following at final concentrations was prepared: 32 mM HEPES-KOH buffer (pH 7.8), 100 mM potassium acetate, 1% dimethyl sulfoxide, 0.11% BSA, 4 mM magnesium acetate, 500 $\mu$M of each dNTP, 1 pmol of the ladder-forming oligonucleotide primer 205RN3(18), primer A12-205R or primer A18-205R (SEQ ID NO:49) as reverse transcription primer, 50 U of RTase M-MLV (Takara Bio) and 1 $\mu$l of one of the template RNAs with varying copy numbers.

The reaction mixture was placed in Thermal Cycler Personal (Takara Bio), incubated at 45°C for 10 minutes, and then cooled to 4°C. After reverse transcription reaction, 15 $\mu$l of a reaction mixture containing the following at final concentrations was added to 10 $\mu$l of the reaction mixture: 32 mM HEPES-KOH buffer (pH 7.8), 100 mM potassium acetate, 1% dimethyl sulfoxide, 0.11% BSA, 4 mM magnesium acetate, 500 $\mu$M of each dNTP, 25 pmol each of primer B134FN3(18) as the second chimeric oligonucleotide primer and primer 205RN3 (18) as the first chimeric oligonucleotide primer, 11 U of BcaBEST (Takara Bio) and 0.05 U of Tli RNase HII. An ICAN reaction was carried out at 55°C using Thermal Cycler Personal (Takara Bio). The amplification products were detected by electrophoresis on a 3% agarose gel.

As a result, the sensitivity of RT-ICAN with reverse transcription using primer 205RN3 was $10^2$ copies. When the ladder-forming oligonucleotide primer A12-205R for the same position having a 12-nucleotide sequence attached to the 5' terminus that anneals to a region upstream (-1 to -12) of the second chimeric oligonucleotide primer or the ladder-forming oligonucleotide primer A18-205R for the same position having an 18-nucleotide sequence attached to the 5' terminus that anneals to a region upstream (-1 to -18) of the second chimeric oligonucleotide primer was used, the sensitivity was $10^1$ copies, i.e., increased by one order of magnitude. In addition, it was confirmed that amplification products in a regular ladder-like pattern resulting from connection through annealing sequences in regions upstream of the primers were obtained by amplification using A12-205R or A18-205R.

As described above, even if the number of nucleotides annealing to a region upstream of the second chimeric oligonucleotide primer was changed, ladder-like amplification through the region occurred, resulting in an increased sensitivity.

Industrial Applicability

The present invention provides an amplification method superior to conventional isothermal nucleic acid amplification methods.

**EP 1 715 037 B1**

Sequence Listing Free Text

SEQ ID NO. 1: A portion of SARS coronavirus genomic RNA reverse transcribed to DNA. "nucleotide 1 to 5 is HindIII restriction site- nucleotide 238 to 242 is BamHI restriction site."

SEQ ID NO. 2: Designed chimeric oligonucleotide primer designated as 205RN3(18) for synthesizing cDNA from mRNA, and to amplify a portion of SARS coronavirus genome. "nucleotides 16 to 18 are ribonucleotides- other nucleotides are deoxyribonucleotides."

SEQ ID NO. 3: Designed oligonucleotide primer designated as A12-205R for synthesizing cDNA from mRNA.

SEQ ID NO. 4: Designed oligonucleotide primer designated as 215R for synthesizing cDNA from mRNA.

SEQ ID NO. 5: Designed oligonucleotide primer designated as A12-215R for synthesizing cDNA from mRNA, and to amplify a portion of SARS coronavirus genome.

SEQ ID NO. 6: Designed oligonucleotide primer designated as A12-223R for synthesizing cDNA from mRNA, and to amplify a portion of SARS coronavirus genome.

SEQ ID NO. 7: Designed chimeric oligonucleotide primer designated as 134FN3(18) to amplify a portion of SARS coronavirus genome. "nucleotides 16 to 18 are ribonucleotides- other nucleotides are deoxyribonucleotides."

SEQ ID NO. 8: Designed oligonucleotide primer designated as A12(-10)-215R for synthesizing cDNA from mRNA, and to amplify a portion of SARS coronavirus genome.

SEQ ID NO. 9: Designed oligonucleotide primer designated as A12(-20)-215R for synthesizing cDNA from mRNA, and to amplify a portion of SARS coronavirus genome.

SEQ ID NO. 10: Designed oligonucleotide primer designated as A12(6)-215R for synthesizing cDNA from mRNA, and to amplify a portion of SARS coronavirus genome.

SEQ ID NO. 11: Designed oligonucleotide primer designated as A12(12)-215R for synthesizing cDNA from mRNA, and to amplify a portion of SARS coronavirus genome.

SEQ ID NO. 12: Designed chimeric oligonucleotide primer designated as B134FN3(16) to amplify a portion of SARS coronavirus genome. "nucleotides 14 to 16 are ribonucleotides- other nucleotides are deoxyribonucleotides." "5'-end is labeled with biotin."

SEQ ID NO. 13: Designed chimeric oligonucleotide primer designated as 205RN3(16) to amplify a portion of SARS coronavirus genome. "nucleotides 14 to 16 are ribonucleotides- other nucleotides are deoxyribonucleotides."

SEQ ID NO. 14: Designed oligonucleotide primer designated as A6(-10)-215R for synthesizing cDNA from mRNA, and to amplify a portion of SARS coronavirus genome.

SEQ ID NO. 15: Designed oligonucleotide primer designated as A9(-10)-215R for synthesizing cDNA from mRNA, and to amplify a portion of SARS coronavirus genome.

SEQ ID NO. 16: Designed oligonucleotide probe designated as SARS-BNI-B for detecting an amplified a portion of SARS coronavirus genome. "5'-end is labeled with FITC."

SEQ ID NO. 17: Designed chimeric oligonucleotide primer designated as 160FN3 to amplify a portion of SARS coronavirus genome. "nucleotides 16 to 18 are ribonucleotides- other nucleotides are deoxyribonucleotides."

SEQ ID NO. 18: Designed chimeric oligonucleotide primer designated as 241RN3 to amplify a portion of SARS coronavirus genome. "nucleotides 12 to 14 are ribonucleotides- other nucleotides are deoxyribonucleotides."

SEQ ID NO. 19: Designed chimeric oligonucleotide primer designated as (A12)241RN3 to amplify a portion of SARS coronavirus genome. "nucleotides 18 to 21 are ribonucleotides- other nucleotides are deoxyribonucleotides."

SEQ ID NO. 20: Designed chimeric oligonucleotide primer designated as 134FN3(16) to amplify a portion of SARS coronavirus genome. "nucleotides 14 to 16 are ribonucleotides- other nucleotides are deoxyribonucleotides."

SEQ ID NO. 21: Designed chimeric oligonucleotide primer designated as ICAN-ALDH2-F to amplify a portion of human aldehyde dehydrogenase 2 gene. "nucleotides 18 to 20 are ribonucleotides- other nucleotides are deoxyribonucleotides."

SEQ ID NO. 22: Designed chimeric oligonucleotide primer designated as ICAN-ALDH2-R to amplify a portion of human aldehyde dehydrogenase 2 gene. "nucleotides 18 to 20 are ribonucleotides- other nucleotides are deoxyribonucleotides."

SEQ ID NO. 23: Designed chimeric oligonucleotide probe designated as ALDH2 wG probe for detecting an amplified a portion of native human aldehyde dehydrogenase 2 gene. "nucleotides 11 is ribonucleotide- other nucleotides are deoxyribonucleotides." "5'-end is labeled with ROX, and 3'-end is labeled with Eclipse."

SEQ ID NO. 24: Designed chimeric oligonucleotide probe designated as ALDH2 mA probe for detecting an amplified a portion of mutant human aldehyde dehydrogenase 2 gene. "nucleotides 11 is ribonucleotide- other nucleotides are deoxyribonucleotides." "5'-end is labeled with FAM, and 3'-end is labeled with Eclipse."

SEQ ID NO. 25: Designed oligonucleotide primer designated as ALDH2-TH1 to amplify a portion of human aldehyde dehydrogenase 2 gene.

SEQ ID NO. 26: Designed oligonucleotide primer designated as ALDH2-TH2 to amplify a portion of human aldehyde dehydrogenase 2 gene.

SEQ ID NO. 27: Designed oligonucleotide primer designated as ALDH2-TH3 to amplify a portion of human aldehyde dehydrogenase 2 gene.

SEQ ID NO. 28: Designed oligonucleotide PCR primer designated as ALDH2-F to amplify a portion of human aldehyde dehydrogenase 2 gene.

SEQ ID NO. 29: Designed oligonucleotide PCR primer designated as ALDH2-R to amplify a portion of human aldehyde dehydrogenase 2 gene.

SEQ ID NO. 30: Designed oligonucleotide primer designated as ALDH2-TH4 to amplify a portion of human aldehyde dehydrogenase 2 gene.

SEQ ID NO. 31: Designed chimeric oligonucleotide primer designated as F2 to amplify a portion of Legionella pneumophila mip gene. "nucleotides 15 to 17 are ribonucleotides- other nucleotides are deoxyribonucleotides."

SEQ ID NO. 32: Designed chimeric oligonucleotide primer designated as R2 to amplify a portion of Legionella pneumophila mip gene. "nucleotides 15 to 17 are ribonucleotides- other nucleotides are deoxyribonucleotides."

SEQ ID NO. 33: Designed chimeric oligonucleotide probe designated as Mip4g12 probe for detecting an amplified a portion of Legionella pneumophila mip gene. "nucleotides 4 is ribonucleotide- other nucleotides are deoxyribonucleotides." "5'-end is labeled with FAM, and 3'-end is labeled with Eclipse."

SEQ ID NO. 34: Designed oligonucleotide primer designated as R2(-13) to amplify a portion of Legionella pneumophila mip gene.

SEQ ID NO. 35: Designed oligonucleotide primer designated as R2(-13)A12-1 to amplify a portion of Legionella pneumophila mip gene.

SEQ ID NO. 36: Designed oligonucleotide primer designated as R2(-13)A12-2 to amplify a portion of Legionella pneumophila mip gene.

SEQ ID NO. 37: Designed oligonucleotide PCR primer designated as c-Ki-ras/12F to amplify a portion of human c-Ki-ras2 gene.

SEQ ID NO. 38: Designed oligonucleotide PCR primer designated as rasT1R to amplify a portion of human c-Ki-ras2 gene.

SEQ ID NO. 39: Designed oligonucleotide PCR primer designated as rasT14F to amplify a portion of human c-Ki-ras2 gene.

SEQ ID NO. 40: Designed oligonucleotide PCR primer designated as rasT4R to amplify a portion of human c-Ki-ras2 gene.

SEQ ID NO. 41: Designed chimeric oligonucleotide primer designated as c-Ki-ras/12FN3 to amplify a portion of human c-Ki-ras2 gene. "nucleotides 18 to 20 are ribonucleotide- other nucleotides are deoxyribonucleotides."

SEQ ID NO. 42: Designed chimeric oligonucleotide primer designated as c-Ki-ras/12RN3 to amplify a portion of human human c-Ki-ras2 gene. "nucleotides 18 to 20 are ribonucleotide- other nucleotides are deoxyribonucleotides."

SEQ ID NO. 43: Designed oligonucleotide primer designated as PJDBF to amplify a portion of Neisseria gonorrhoeae cppB gene.

SEQ ID NO. 44: Designed oligonucleotide primer designated as PJDBR to amplify a portion of Neisseria gonorrhoeae cppB gene.

SEQ ID NO. 45: Designed chimeric oligonucleotide primer designated as PJDB0FN3 to amplify a portion of Neisseria gonorrhoeae cppB gene. "nucleotides 18 to 20 are ribonucleotide- other nucleotides are deoxyribonucleotides."

SEQ ID NO. 46: Designed chimeric oligonucleotide primer designated as PJDB0RN3 to amplify a portion of Neisseria gonorrhoeae cppB gene. "nucleotides 15 to 17 are ribonucleotide- other nucleotides are deoxyribonucleotides."

SEQ ID NO. 47: Designed oligonucleotide primer designated as A6-215R to amplify a portion of SARS coronavirus genome.

SEQ ID NO. 48: Designed oligonucleotide primer designated as A9-215R to amplify a portion of SARS coronavirus genome.

SEQ ID NO. 49: Designed oligonucleotide primer designated as A18-205R to amplify a portion of SARS coronavirus genome.

## Claims

1. A method for amplifying a nucleic acid, the method comprising the steps of:

    (A) preparing a reaction mixture selected from:

        (a) a nucleic acid as a template, a deoxyribonucleotide triphosphate, a DNA polymerase having a strand displacement activity, at least two chimeric oligonucleotide primers, at least one ladder-forming oligonucleotide primer and an RNase H; or
        (b) a nucleic acid as a template, a deoxyribonucleotide triphosphate, a DNA polymerase having a strand displacement activity, at least two chimeric oligonucleotide primers and an RNase H, wherein one of the chimeric oligonucleotide primers serves as a ladder-forming oligonucleotide primer,
        wherein each chimeric oligonucleotide primer is a primer that contains a ribonucleotide as well as at least one selected from the group consisting of a deoxyribonucleotide and a nucleotide analog, and the ribonucleotide is positioned at the 3' terminus or on the 3'-terminal side of the primer,
        wherein the chimeric oligonucleotide primers comprise at least a first chimeric oligonucleotide primer which is complementary to a nucleotide sequence of the nucleic acid as a template and a second chimeric oligonucleotide primer which is homologous to a nucleotide sequence of the nucleic acid as a template, and
        wherein the ladder-forming oligonucleotide primer has a sequence complementary to a region of the nucleic acid as a template that is complementary to the first chimeric oligonucleotide primer and/or a nucleotide sequence 3' to said region, and has, on its 5' side, a sequence complementary to: a nucleotide sequence on the 5' side of the second chimeric oligonucleotide primer which is homologous to the nucleic acid as a template; a nucleotide sequence of the nucleic acid as a template corresponding to a region 5' to the 5' terminus of the portion homologous to the second chimeric oligonucleotide primer; or both; and

    (B) incubating the reaction mixture for a sufficient time to generate a ladder-like amplification product under constant-temperature conditions under which specific annealing of the primer to the nucleic acid as a template, a reaction of synthesizing an extended strand and a strand displacement reaction by the DNA polymerase, as well as a reaction of cleaving an extended strand by the RNase H take place.

2. The method according to claim 1, wherein the nucleic acid as a template is an RNA, and the nucleic acid is treated beforehand with a deoxyribonucleotide triphosphate, a DNA polymerase having a reverse transcription activity and at least one ladder-forming oligonucleotide primer to convert the nucleic acid into a reverse transcription product.

3. The method according to claim 1, wherein the reaction mixture in step (A) further contains a DNA polymerase having a reverse transcription activity.

4. The method according to claim 2 or 3, wherein the nucleic acid as a template is an mRNA.

5. The method according to claim 2 or 3, wherein a single DNA polymerase having a reverse transcription activity and a strand displacement activity serves as the DNA polymerase having a reverse transcription activity and the DNA polymerase having a strand displacement activity.

6. A method for detecting a target nucleic acid, the method comprising the steps of:

(a) amplifying a target nucleic acid according to the method for amplifying a nucleic acid defined by claim 1; and
(b) detecting the target nucleic acid amplified in the above step.

**Patentansprüche**

1. Verfahren zum Amplifizieren einer Nukleinsäure, wobei das Verfahren die Schritte umfasst:

(A) Herstellen eines Reaktionsgemisches, das ausgewählt ist aus:

(a) eine Nukleinsäure als eine Matrize, ein Desoxyribonukleotidtriphosphat, eine DNA-Polymerase mit Strangverdrängungsaktivität, mindestens zwei chimäre Oligonukleotid-Primer, mindestens ein Leiter-bildender Oligonukleotid-Primer und eine RNase H, oder
(b) eine Nukleinsäure als eine Matrize, ein Desoxyribonukleotidtriphosphat, eine DNA-Polymerase mit Strangverdrängungsaktivität, mindestens zwei chimäre Oligonukleotid-Primer und eine RNase H, wobei einer der chimären Oligonukleotid-Primer als ein Leiter-bildender Oligonukleotid-Primer dient,
wobei jeder chimäre Oligonukleotid-Primer ein Primer ist, der ein Ribonukleotid, sowie mindestens ein Mitglied aus der Gruppe, bestehend aus einem Desoxyribonukleotid und einem Nukleotid-Analogon, enthält und das Ribonukleotid am 3'-Terminus oder auf der 3'-terminalen Seite des Primers liegt,
wobei die chimären Oligonukleotid-Primer mindestens einen ersten chimären Oligonukleotid-Primer, der zu einer Nukleotidsequenz der Nukleinsäure als eine Matrize komplementär ist, und einen zweiten chimären Oligonukleotid-Primer, der zu einer Nukleotidsequenz der Nukleinsäure als eine Matrize homolog ist, umfassen, und
wobei der Leiter-bildende Oligonukleotid-Primer eine Sequenz aufweist, die komplementär ist zu einer Region der Nukleinsäure als eine Matrize, die zu dem ersten chimären Oligonukleotid-Primer komplementär ist, und/oder einer Nukleotidsequenz 3' zu dieser Region, und auf seiner 5'-Seite eine Sequenz aufweist, die komplementär ist zu: einer Nukleotidsequenz auf der 5'-Seite des zweiten chimären Oligonukleotid-Primers, die zu der Nukleinsäure als eine Matrize homolog ist, einer Nukleotidsequenz der Nukleinsäure als eine Matrize, die einer Region 5' zu dem 5'-Terminus des Teils, der zu dem zweiten chimären Oligonukleotid-Primer homolog ist, entspricht, oder beides, und

(B) Inkubieren des Reaktionsgemisches für einen ausreichenden Zeitraum, um ein Leiter-ähnliches Amplifikationsprodukt unter konstanten Temperaturbedingungen zu erzeugen, unter denen ein spezifisches Anlagern des Primers an die Nukleinsäure als eine Matrize, eine Synthesereaktion eines verlängerten Strangs und eine Strangverdrängungsreaktion durch die DNA-Polymerase, sowie eine Spaltungsreaktion eines verlängerten Strangs durch die RNase H stattfinden.

2. Verfahren gemäß Anspruch 1, wobei die Nukleinsäure als eine Matrize eine RNA ist und die Nukleinsäure zuvor mit einem Desoxyribonukleotidtriphosphat, einer DNA-Polymerase mit reverser Transkriptionsaktivität und mindestens einem Leiter-bildenden Oligonukleotid-Primer behandelt wird, um die Nukleinsäure in ein reverses Transkriptionsprodukt umzuwandeln.

3. Verfahren gemäß Anspruch 1, wobei das Reaktionsgemisch in Schritt (A) ferner eine DNA-Polymerase mit reverser Transkriptionsaktivität enthält.

4. Verfahren gemäß Anspruch 2 oder 3, wobei die Nukleinsäure als eine Matrize eine mRNA ist.

5. Verfahren gemäß Anspruch 2 oder 3, wobei eine einzige DNA-Polymerase mit reverser Transkriptionsaktivität und einer Strangverdrängungsaktivität als die DNA-Polymerase mit reverser Transkriptionsaktivität und die DNA-Polymerase mit einer Strangverdrängungsaktivität dient.

6. Verfahren zum Nachweis einer Ziel-Nukleinsäure, wobei das Verfahren die Schritte umfasst:

(a) Amplifizieren einer Ziel-Nukleinsäure gemäß dem Verfahren zum Amplifizieren einer Nukleinsäure, das durch Anspruch 1 definiert ist, und

(b) Nachweis der Ziel-Nukleinsäure, die in dem vorstehenden Schritt amplifiziert wurde.

**Revendications**

1. Méthode pour amplifier un acide nucléique, la méthode comprenant les étapes de :

   A) préparation d'un mélange réactionnel choisi parmi :

   a) un acide nucléique servant de matrice, un désoxyribonucléotide triphosphate, une ADN polymérase ayant une activité de déplacement de brin, au moins deux amorces oligonucléotidiques chimères, au moins une amorce oligonucléotidique formant une échelle et une RNase H ; ou
   b) un acide nucléique servant de matrice, un désoxyribonucléotide triphosphate, une ADN polymérase ayant une activité de déplacement de brin, au moins deux amorces oligonucléotidiques chimères et une RNase H,
   dans laquelle une des amorces oligonucléotidiques chimères sert d'amorce oligonucléotidique formant une échelle ;
   dans laquelle chaque amorce oligonucléotidique chimère est une amorce qui contient un ribonucléotide ainsi qu'au moins un membre choisi dans le groupe constitué d'un désoxyribonucléotide et d'un analogue de nucléotide, et le ribonucléotide est placé à l'extrémité 3' ou sur le côté 3' terminal de l'amorce,
   dans laquelle les amorces oligonucléotides chimères comprennent au moins une première amorce oligonucléotidique chimère qui est complémentaire d'une séquence nucléotidique de l'acide nucléique servant de matrice et une seconde amorce oligonucléotidique chimère qui est homologue d'une séquence nucléotidique de l'acide nucléique servant de matrice, et ;
   dans laquelle l'amorce oligonucléotidique formant une échelle a une séquence complémentaire d'une région de l'acide nucléique servant de matrice qui est complémentaire de la première amorce oligonucléotidique chimère et/ou d'une séquence nucléotidique en 3' par rapport à ladite région, et a, sur son côté 5', une séquence complémentaire d'une séquence nucléotidique sur le côté 5' de la seconde amorce oligonucléotidique chimère qui est homologue de l'acide nucléique servant de matrice ; une séquence nucléotidique de l'acide nucléique servant de matrice correspondant à une région 5' par rapport à l'extrémité 5' de la portion homologue de la seconde amorce oligonucléotidique chimère ; ou les deux ; et

   (B) l'incubation du mélange réactionnel pendant un temps suffisant pour obtenir un produit d'amplification de type échelle dans des conditions de température constante dans lesquelles l'hybridation spécifique de l'amorce avec l'acide nucléique servant de matrice, une réaction de synthèse d'un brin prolongé et une réaction de déplacement de brin par l'ADN polymérase, ainsi qu'une réaction de clivage d'un brin prolongé par la RNase H ont lieu.

2. Méthode selon la revendication 1, dans laquelle l'acide nucléique servant de matrice est un ARN, et l'acide nucléique est traité d'abord avec un désoxyribonucléotide triphosphate, une ADN polymérase ayant une activité de rétrotranscription et au moins une amorce oligonucléotidique formant une échelle pour convertir l'acide nucléique en un produit de rétrotranscription.

3. Méthode de la revendication 1, dans laquelle le mélange réactionnel de l'étape (A) contient en outre une ADN polymérase ayant une activité de rétrotranscription.

4. Méthode selon la revendication 2 ou 3, dans laquelle l'acide nucléique servant de matrice est un ARNm.

5. Méthode selon la revendication 2 ou 3, dans laquelle une seule ADN polymérase ayant une activité de rétrotranscription et une activité de déplacement de brin est utilisée comme l'ADN polymérase ayant une activité de rétrotranscription et l'ADN polymérase ayant une activité de déplacement de brin.

6. Méthode pour détecter un acide nucléique cible, la méthode comprenant les étapes :

   (a) d'amplification d'un acide nucléique cible selon la méthode destinée à amplifier un acide nucléique définie par la revendication 1 ; et
   (b) de détection de l'acide nucléique cible amplifié dans l'étape ci-dessus.

Figure 1

Figure 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 7114718 B
- WO 9719193 A
- WO 0028082 A
- WO 0056877 A
- US 5824517 A
- WO 9909211 A
- WO 9525180 A

- WO 9949081 A
- WO 9503426 A
- JP 2003052380 A
- WO 0222831 A
- JP 2978001 B
- JP 2003412326 A

**Non-patent literature cited in the description**

- **Hafner, G.J. et al.** *BioTechniques,* 2001, vol. 30, 852-867

- Molecular Cloning: A Laboratory Manual. Cold Spring Harbor Laboratory, 1989